# EUROPÄISCHE PATENTANMELDUNG

(11) **EP 2 258 375 A1**
(43) Veröffentlichungstag der Anmeldung: **08.12.2010**
(21) Anmeldenummer: 09075249.4
(22) Anmeldetag: 04.06.2009
(51) Int. Cl.: A61K 31/56, C07J 3/00, A61P 5/32

(54) **17B-alkyl-17alpha-oxy-estratriene**

(71) Anmelder: Bayer Schering Pharma Aktiengesellschaft, 13353 Berlin (DE)
(72) Erfinder: Bohlmann, Rolf Dr., 14055 Berlin (DE); Heinrich, Nikolaus Dr., 12159 Berlin (DE); Hübner, Jan Dr., 10317 Berlin (DE); Kettschau, Georg Dr., 13187 Berlin (DE); Künzer, Hermann Dr., 13347 Berlin (DE); Lienau, Philip Dr., 10997 Berlin (DE); Gerisch, Michael Dr., 42329 Wuppertal (DE); Köhr, Silke Dr., 12359 Berlin (DE); Lang, Dieter Dr., 42553 Velbert (DE); Denner, Karsten Dr., 13156 Berlin (DE); Sander, Michael Dr., 50226 Frenchen (DE); Hoffmann, Jens Dr., 16567 Mühlenbeck (DE); Wintermantel, Tim Dr., 50670 Köln (DE)

(57) **Zusammenfassung**

Die Erfindung betrifft 17β-Alkyl-17α-oxy-estratriene der Formel (I), Verfahren zu deren Herstellung, Verwendung der 17β-Alkyl-17α-oxy-estratriene zur Herstellung von Arzneimitteln sowie diese Verbindungen enthaltende pharmazeutische Präparate.

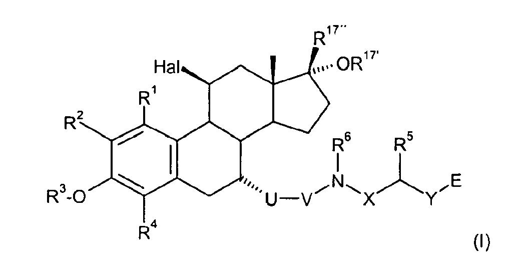

## Beschreibung

Die Erfindung betrifft 17β-Alkyl-17α-oxy-estratriene und Verfahren zu deren Herstellung, Verwendung der 17β-Alkyl-17α-oxy-estratriene zur Herstellung von Arzneimitteln sowie diese Verbindungen enthaltende pharmazeutische Präparate.

Die erfindungsgemässen Verbindungen weisen antiestrogene Wirksamkeit auf, d.h. diese Stoffe üben Hemmwirkungen gegenüber Estrogenen aus. Derartige Stoffe sind bereits zahlreich beschrieben worden.

Beispielsweise sind antiestrogen-wirkende Verbindungen aus EP 0 138 504 B1 bekannt. Es handelt sich danach im wesentlichen um Estra-1,3,5(10)-trien-Derivate, die in 3-Stellung u.a. mit Hydroxy oder Alkoxy, in 17α-Stellung mit Hydroxy und in 17β-Stellung u.a. mit Wasserstoff oder Alkyl substituiert sind. Diese Verbindungen weisen in 7α-Stellung ferner eine Alkyl-Seitenkette auf, die teilweise fluoriert sein kann und die u.a. durch Amido-, Amino-, Amin-N-oxid-, Oxy-, Sulfanyl-, Sulfinyl- und/oder Sulfonylgruppen unterbrochen sein kann.

In WO 99/33855 A1 sind 11β-Halogen-7α-substituierte Estra-1,3,5(10)-triene beschrieben. die in 3- und in 17-Stellung Hydroxygruppen aufweisen können. Die 7α-Seitenkette ist eine teilweise fluorierte, gegebenenfalls ungesättigte Kohlenwasserstoffkette, die durch ein AminStickstoffatom oder eine Sulfanyl-, Sulfinyl- oder Sulfonylgruppe unterbrochen ist.

Weitere Verbindungen sind in WO 98/07740 A1 beschrieben. Es handelt sich hierbei um substituierte 7α-(ξ-Aminoalkyl)-estra-1,3,5(10)-triene. Diese Verbindungen weisen in 3-Stellung vorzugsweise eine Hydroxy-, Methoxy- oder Acetyloxygruppe und in 17β- und/oder 17α-Stellung vorzugsweise eine Methyl- bzw. Trifluormethylgruppe auf. In 11 β-Stellung ist vorzugsweise ein Fluoratom vorgesehen und in 7α-Stellung eine Alkyl-Seitenkette, die endständig zumindest teilweise fluoriert ist und durch ein Amin-N-Atom und durch eine Sulfanyl-, Sulfinyl- oder Sulfonylgruppe unterbrochen ist.

In WO 97/45441 A1 sind 7α-(5-Methylaminopentyl)-estra-1,3,5(10)-triene offenbart, die in 3-Stellung und in 17α-Stellung eine Hydroxygruppe aufweisen. In 17β-Stellung kann eine Methyl- oder Ethinylgruppe vorgesehen sein. Ferner kann das Estratriengrundgerüst auch in 2-Stellung mit einem Fluoratom substituiert sein.

Es hat sich herausgestellt, dass die bekannten Verbindungen bei der Applikation eine Vielfalt biologisch sehr aktiver Metaboliten bilden. Die Bildung dieser Metaboliten führt zu unerwünschten Wirkungen und damit zu einem unkontrollierbaren Wirkungsspektrum. Insbesondere können sich Nebenwirkungen einstellen, oder die gewünschte Primärwirkung (antiestrogene Wirkung) wird durch spontane Bildung dieser Metaboliten unkontrollierbar. Ausserdem ist die Verträglichkeit der bekannten Verbindungen bei oraler Applikation nicht befriedigend. Es hat sich insbesondere herausgestellt, dass die bekannten Verbindungen die Anreicherung von alveolaren Makrophagen begünstigen.

Allen Antiestrogenen des Standes der Technik gemeinsam ist eine unbefriedigende orale Bioverfügbarkeit. Außerdem zeigen die Antiestrogene des Standes der Technik Wechselwirkungen mit Cytochrom-P450-Oxidasen.

Den strukturell nächstliegenden Stand der Technik bilden Verbindungen der WO 03/045972, die sich von den erfindungsgemäßen Verbindungen dadurch unterscheiden, dass die von Wasserstoff verschiedene Gruppe in Position 17 nicht in α-Position, sondern in β-Position und der ebenfalls in der Position 17 gebundene sauerstoffhaltige Rest nicht in β-Position, sondern in α-Position an das Estratriengrundgerüst gebunden ist.

Ausgehend von diesem Stand der Technik liegt der vorliegenden Erfindung die Aufgabe zugrunde, weitere antiestrogene Verbindungen bereitzustellen, die keine oder nur wenige biologisch aktive Metaboliten bilden. Ausserdem sollen die gesuchten Verbindungen bei oraler Gabe eine verbesserte Bioberfügbarkeit aufweisen, so dass die Belastung des Patienten reduziert ist. Dies erhöht die Patienten-Compliance.

Die erfindungsgemässe Aufgabe wird gelöst durch 17β-Alkyl-17α-oxy-estratriene der allgemeinen Formel I, 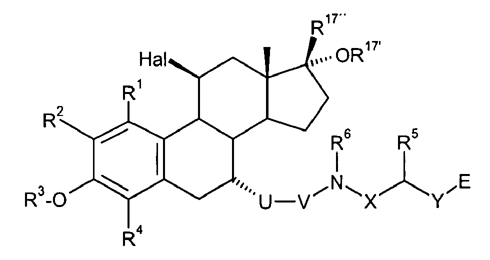 in der
- **Hal**: für Fluor oder Chlor steht, das in 11 β-Position an das Estratriengrundgerüst gebunden ist; und
- **R¹, R² und R⁴**: unabhängig voneinander für Wasserstoff, Fluor, Chlor oder Brom stehen, und
- **R³**: für Wasserstoff oder einen C₁-C₄-Alkyl- oder C₁-C₄-Alkanoylrest steht, und
- **R^{17'}**: für Wasserstoff oder einen C₁-C₄-Alkyl- oder C₁-C₄-Alkanoylrest steht,
- **R^{17"}**: für einen gegebenenfalls ein- oder mehrfach fluorierten C₁-C₄-Alkyl-, C₂-C₄- Alkenyl- oder C₂ - C₄-Alkinylrest steht, wobei **R^{17'}**-O in 17α-Position und **R^{17"}** in 17β-Position an das Estratriengrundgerüst gebunden sind; und
- **U**: für einen gerad- oder verzweigtkettigen C₁-C₁₃-Alkylen-, C₂-C₁₃-Alkenylen- oder C₂-C₁₃-Alkinylenrest steht, oder für die Gruppe **A-B** wobei **A** an das Estratriengrundgerüst gebunden ist und einen über -CH₂- an das Estratriengrundgerüst gebundenen Benzylidenrest, einen Phenylenrest oder einen über die Alkylgruppe an das Estratriengrundgerüst gebundenen C₁-C₃- Alkylen-Phenylenrest darstellt und **B** für einen gerad- oder verzweigtkettigen C₁-C₁₃-Alkylen-, C₂-C₁₃-Alkenylen- oder C₂-C₁₃-Alkinylenrest steht und wobei **A** und **B** auch über ein O-Atom miteinander verbunden sein können,
- **V**: für eine Methylen- oder eine -C(O)-Gruppe steht und
- **X**: für eine Bindung oder eine C₁-C₃-Alkylengruppe steht und
- **R⁵**: für Wasserstoff oder einen C₁-C₄-Alkyl-, C₂-C₄-Alkenyl- oder C₂-C₄-Alkinylrest steht
- **R⁶**: für Wasserstoff oder die Gruppe -CH₂-**R⁷** oder C(O)-**R⁷** steht, worin **R⁷** steht für Wasserstoff oder einen gerad- oder verzweigtkettigen, nichtfluorierten oder zumindest teilweise fluorierten C₁-C₆-Alkyl-, C₂-C₆-Alkenyl- oder C₂-C₆- Alkinylrest, der einfach oder mehrfach mit Hydroxy substituiert sei kann, oder
- **R⁵ und R⁶**: zusammen mit X und dem Stickstoffatom der Seitenkette einen 4 bis 6 gliedrigen Heterocyclylring bilden, der zusätzlich zum Stickstoffatom der Seitenkette ein weiteres Heteroatom aufweisen kann und/oder eine Carbonylgruppe enthalten kann,
- **Y**: für eine C₅-C₈-Alkylengruppe steht,
- **E**: für einen C₁-C₄-Perfluoroalkylrest oder für einen Phenylrest steht, der ein- bis fünffach substituiert ist mit Halogen oder -CF₃,
sowie deren Enantiomere und Diastereomere, deren Salze, Solvate und Salze der Solvate.

Der Anmeldungen liegen folgende Definitionen zugrunde:

### Cₙ-Alkyl:

Cₙ-Alkyl steht für einen linearen oder verzweigten, gesättigten, monovalenten Kohlenwasserstoffrest mit n Kohlenstoffatomen.

### Cₙ-Alkylen

Cₙ-Alkylen steht für einen linearen oder verzweigten, gesättigten, divalenten Kohlenwasserstoffrest mit n Kohlenstoffatomen.

### Cₙ-Alkenylen:

Cₙ-Alkenylen steht für einen linearen oder verzweigten, divalenten Kohlenwasserstoffrest mit n Kohlenstoffatomen und mindestens einer Doppelbindung.

### Cₙ-Alkinylen

Cₙ-Alkinylen steht für einen linearen oder verzweigten, divalenten Kohlenwasserstoffrest mit n Kohlenstoffatomen und mindestens einer Dreifachbindung.

### Cₙ-Alkenyl:

Cₙ-Alkenyl steht für einen linearen oder verzweigten, monovalenten Kohlenwasserstoffrest mit n Kohlenstoffatomen und mindestens einer Doppelbindung.

### Cₙ-Alkinyl:

Cₙ-Alkinyl steht für einen linearen oder verzweigten, monovalenten Kohlenwasserstoffrest mit n Kohlenstoffatomen und und mindestens einer Dreifachbindung.

### Cₙ-Alkylcarbonyl= Cₙ-Alkanoyl

Cₙ-Alkylcarbonyl steht für die Gruppe -C(O)-Cₙ-Alkyl
In der Regel ist n gleich 1 bis 6, bevorzugt 1 bis 4, und besonders bevorzugt 1 bis 3. Beispielhaft und vorzugsweise seien genannt:
Acetyl- und Propanoyl.

### Heteroatome

Unter Heteroatomen sind Sauerstoff-, Stickstoff- oder Schwefel-Atome zu verstehen.

### Heteroaryl

Heteroaryl ist ein monovalentes, aromatisches, mono- oder bicyclisches Ringsystem mit mindestens einem von einem Kohlenstoff verschiedenen Heteroatom. Als Heteroatome können Stickstoffatome, Sauerstoffatome und/oder Schwefelatome vorkommen. Die Bindungsvalenz kann an einem beliebigen aromatischen Kohlenstoffatom oder an einem Stickstoffatom sein.

### Heterocyclyl

Heterocyclyl im Sinne der Erfindung ist ein vollständig oder teilweise hydriertes Heteroaryl (vollständig hydriertes Heteroaryl = gesättigtes Heterocyclyl) d.h. ein nicht aromatisches mono- oder bicyclisches Ringsystem mit mindestens einem von einem Kohlenstoff verschiedenen Heteroatom oder einer Heterogruppe. Als Heteroatome können Stickstoffatome, Sauerstoffatome und/oder Schwefelatome vorkommen. Die Bindungsvalenz kann an einem beliebigen Kohlenstoffatom oder an einem Stickstoffatom sein.

Ein monocyclischer Heteroacyclylring gemäß der vorliegenden Erfindung kann 4 bis 6 Ringatome aufweisen.
Heterocycylring mit 4 Ringatomen (viergleidrig) umfasst beispielsweise Azetidinyl. Heterocycylringe mit 5 Ringatomen (fünfgliedrig) umfassen beispielsweise die Ringe Pyrrolidinyl, Imidazolidinyl, Pyrazolidinyl und Pyrrolinyl.
Heterocyclylringe mit 6 Ringatomen umfassen beispielsweise die Ringe Piperidinyl, Piperazinyl, Morpholinyl und Thiomorpholinyl.

### Halogen

Die Bezeichnung Halogen umfasst Fluor, Chlor, Brom und Iod.
Bevorzugt ist Fluor.

In der allgemeinen Formel (I) kann **Hal** stehen für Fluor oder Chlor, das in 11 β-Position an das Estratriengrundgerüst gebunden ist.
Bevorzugt steht Hal für ein Fluoratom.

In der allgemeinen Formel (I) können **R¹, R² und R⁴** unabhängig voneinander stehen für Wasserstoff, Fluor, Chlor oder Brom stehen.
Bevorzugt stehen **R¹, R² und R⁴** unabhängig voneineanderfür Wasserstoff, Chlor oder Brom. Besonders bevorzugt steht **R¹** für Wasserstoff, **R²** für Wasserstoff oder Chlor und **R⁴** für Wasserstoff Chlor oder Brom.
Außerordentlich bevorzugt stehen R¹, R² und R³ für Wasserstoff.

In der allgemeinen Formel (I) kann **R³** stehen für Wasserstoff oder einen C₁-C₄-Alkyl- oder C₁-C₄-Alkanoylrest.
Bevorzugt steht **R³** für Wasserstoff, Methyl, Ethyl, n-Propyl, iso-Propyl, n-Butyl, iso-Butyl oder *tert*-Butyl oder ein entsprechendes Alkanyol (Acetyl, Propionyl, Butanoyl).
Besonders bevorzugt steht **R³** für Wasserstoff, einen Methyl- oder Acetylrest. Außerordentlich bevorzugt steht **R³** für Wasserstoff.

In der allgemeinen Formel (I) kann **R⁵** stehen für Wasserstoff oder einen C₁-C₄-Alkyl-, C₂-C₄-Alkenyl- oder C₂-C₄-Alkinylrest.
**R⁵** steht vorzugsweise für Wasserstoff oder einen C₁-C₃-Alkylrest.
**R⁵** steht besonders bevorzugt für Wasserstoff.

In der allgemeinen Formel (I) kann **R⁶** für Wasserstoff oder die Gruppe -CH₂-**R⁷** oder C(O)-**R⁷**, worin **R⁷** steht für Wasserstoff oder einen gerad- oder verzweigtkettigen, nichtfluorierten oder zumindest teilweise fluorierten C₁-C₆-Alkyl-, C₂-C₆-Alkenyl- oder C₂-C₆-Alkinylrest, der einfach oder mehrfach mit Hydroxy substituiert sei kann, oder
**R⁶** steht vorzugsweise für Wasserstoff oder -CH₂-**R⁷**, worin **R⁷** insbesondere für Wasserstoff oder einen Methyl- oder Ethylrest steht.
**R⁶** steht besonders bevorzugt eine Methylgruppe.

In der allgemeinen Formel (I) können **R⁵** und **R⁶** alternativ zusammen mit **X** und dem Stickstoffatom der Seitenkette einen 4 bis 6 gliedrigen Heterocyclylring bilden, der zusätzlich zum Stickstoffatom der Seitenkette ein weiteres Heteroatom aufweisen kann und/oder eine Carbonylgruppe enthalten kann.
**R⁵** und **R⁶** bilden zusammen mit dem Stickstoffatom der Seitenkette bevorzugt einen 5-gliedrigen Heterocyclylring, der zusätzlich zum Stickstoffatom der Seitenkette ein weiteres Heteroatom aufweisen kann und/oder eine Carbonylgruppe enthalten kann.
**R⁵ und R⁶** bilden zusammen mit dem Stickstoffatom der Seitenkette besonders bevorzugt einen Pyrrolidinring.

In der allgemeinen Formel (I) kann **R^{17'}** stehen für Wasserstoff oder einen C₁-C₄-Alkyl- oder C₁-C₄-Alkanoylrest.

In der allgemeinen Formel (I) kann **R^{17"}** stehen für einen gegebenenfalls ein- oder mehrfach fluorierten C₁-C₄-Alkyl-, C₂-C₄-Alkenyl- oder C₂-C₄-Alkinylrest.
**R^{17'}**-O ist in 17α-Position und **R^{17"}** in 17β-Position an das Estratriengrundgerüst gebunden. **R^{71'}** und **R^{17"}** sind insbesondere Methyl, Ethyl, n-Propyl, iso-Propyl, n-Butyl, iso-Butyl und *tert*-Butyl.
Für **R^{17'}** bevorzugt ist zusätzlich Wasserstoff, Acetyl, Propionyl und Butanoyl.
Für **R^{17"}** bevorzugt ist ausserdem Ethinyl, 1-Propinyl, 2-Propinyl, 1-Butinyl, 2-Butinyl und 3-Butinyl, sowie Trifluormethyl, Pentafluorethyl, Heptafluorpropyl und Nonafluorbutyl.
**R^{17'}** steht besonders bevorzugt für Wasserstoff, einen Methylrest oder Acetylrest.
**R^{17"}** steht besonders bevorzugt einen Methyl-, Ethinyl- oder Trifluormethylrest.
**R^{17'}** steht außerordentlich bevorzugt für Wasserstoff.
**R^{17"}** steht außerordentlich bevorzugt einen Methylrest.

In der allgemeinen Formel (I) kann **U** stehen für einen gerad- oder verzweigtkettigen C₁-C₁₃-Alkylen-, C₂-C₁₃-Alkenylen- oder C₂-C₁₃-Alkinylenrest steht, oder
für die Gruppe **A-B** wobei
**A** an das Estratriengrundgerüst gebunden ist und einen über -CH₂- an das Estratriengrundgerüst gebundenen Benzylidenrest, einen Phenylenrest oder einen über die Alkylgruppe an das Estratriengrundgerüst gebundenen C₁-C₃-Alkylen-Phenylenrest darstellt und
B für einen gerad- oder verzweigtkettigen C₁-C₁₃-Alkylen-, C₂-C₁₃-Alkenylen- oder C₂-C₁₃-Alkinylenrest steht und
wobei **A** und **B** auch über ein O-Atom miteinander verbunden sein können.
**U** kann insbesondere ein gerad- oder verzweigtkettiger Alkylenrest sein.
Bevorzugt ist ein Methylen-, Ethylen-, Propylen-, Butylen-, Pentylen-, Hexylen-, Heptylen-, Octylen-, Nonylen-, Decylen-, Undecylen-, Dodecylen- oder Tridecylenrest.
Besonders bevorzugt steht **U** für -(CH₂)ₚ₋, wobei p eine ganze Zahl von 2 bis 10 ist. Insbesondere bevorzugt ist **U** ein Butylen-, Pentylen-, Hexylen- oder Heptylenrest. Außerordentlich bevorzugt ist **U** ein n-Butylenrest, d.h. in der Formel -(CH₂)ₚ- für **U** ist p = 4.

In der allgemeinen Formel (I) kann **V** stehen für eine Methylen- oder eine C(O)-Gruppe.
**V** steht insbesondere für eine Methylengruppe. Damit kann die Gruppierung **U-V** in einer außerordentlich bevorzugten Ausführungsform n-Pentylen sein.

In der allgemeinen Formel (I) kann **X** stehen für eine Bindung oder eine C₁-C₃-Alkylengruppe. Bevorzugt für **X** ist eine Bindung oder eine Methylengruppe.
Besonders bevorzugt für **X** ist eine Bindung.

In der allgemeinen Formel (I) kann **Y** stehen für eine C₅-C₈-Alkylengruppe.
Bevorzugt für **Y** ist eine Bindung oder eine C₅-C₇-Alkylengruppe.
Besonders bevorzugt für **Y** ist eine n-Pentylen- oder n-Hexylengruppe.

In der allgemeinen Formel (I) kann **E** stehen für einen C₁-C₄-Perfluoroalkylrest oder für einen Phenylrest, der ein- bis fünffach substituiert ist mit Halogen oder -CF₃,
**E** steht bevorzugt für -CF₃, -C₂F₅, -C₃F₇, -C₄F₉, für einen Phenyl, der ein- bis dreifach substituiert ist mit Halogen und/oder Trifluormethyl.
**E** steht besonders bevorzugt für -C₂F₅, -C₃F₇, -C₄F₉ oder für einen Trifluormethylphenylrest. **E** steht außerordentlich bevorzugt für -C₂F₅.

Eine bevorzugte Untergruppe von Verbindungen bilden Verbindungen gemäß Formel (I), in der
- **Hal**: für Fluor steht, und
- **R¹, R² und R⁴**: unabhängig voneinanderfür Wasserstoff, Chlor oder Brom stehen, und
- **R³**: für Wasserstoff, einen Methyl- oder Acetylrest steht, und
- **R⁵**: für Wasserstoff oder einen C₁-C₃-Alkylrest steht, und
- **R⁶**: für Wasserstoff oder -CH₂-**R⁷** steht, worin **R⁷** für Wasserstoff oder einen Methyl- oder Ethylrest steht, oder
- **R⁵ und R⁶**: zusammen mit dem Stickstoffatom der Seitenkette einen 5-gliedrigen Heterocyclylring bilden, der zusätzlich zum Stickstoffatom der Seitenkette ein weiteres Heteroatom aufweisen kann und/oder eine Carbonylgruppe enthalten kann, und
- **R^{17'}**: für Wasserstoff, einen Methylrest oder Acetylrest steht, und
- **R^{17"}**: für einen Methyl-, Ethinyl- oder Trifluormethylrest steht, und
- **U**: für einen Butylen-, Pentylen-, Hexylen- oder Heptylenrest steht, und
- **V**: für eine Methylengruppe steht, und
- X: für eine Bindung oder eine Methylengruppe steht, und
- **Y**: für eine Bindung oder eine C₅-C₇-Alkylengruppe steht, und
- **E**: für -C₂F₅, -C₃F₇, -C₄F₉ oder für einen Trifluormethylphenylrest steht,
sowie deren Enantiomere und Diastereomere, deren Salze, Solvate und Salze der Solvate.

Eine außerordentlich bevorzugte Untergruppe von Verbindungen bilden Verbindungen gemäß Formel (I), in der
- **R¹**: für Wasserstoff steht, und
- **R²**: für Wasserstoff oder Chlor steht, und
- **R⁴**: für Wasserstoff, Chlor oder Brom steht, und
- **R³**: für Wasserstoff steht, und
- **Hal**: für Fluor steht, und
- **R⁵**: für Wasserstoff und **R⁶** für eine Methylgruppe steht oder
- **R⁵ und R⁶**: zusammen mit dem Stickstoffatom der Seitenkette einen Pyrrolidinring bilden, und
- **R^{17'}**: für Wasserstoff steht, und
- **R^{17"}**: für einen Methylrest steht, und
- **U**: für einen n-Butylenrest steht, und
- **V**: für eine Methylengruppe steht, und
- **X**: für eine Bindung steht, und
- **Y**: für eine n-Pentylen- oder n-Hexylengruppe steht, und
- **E**: für -C₂F₅ steht,
sowie deren Enantiomere und Diastereomere, deren Salze, Solvate und Salze der Solvate.

Gemäß der vorliegenden Erfindung sind auch pharmakologisch verträgliche Säureadditionssalze sowie Ester der 17β-Alkyl-17α-oxy-estratriene umfasst. Bei den Additionssalzen handelt es sich um die entsprechenden Salze mit anorganischen und organischen Säuren. Als Additionssalze kommen insbesondere die Hydrochloride, Hydrobromide, Acetate, Citrate, Oxalate, Tartrate und Methansulfonate in Betracht. Falls **R³** und **R^{17'}** Wasserstoff sind, so dass ein 3,17α-Diol vorliegt, können auch die Ester dieser Hydroxyverbindungen gebildet werden. Diese Ester werden vorzugsweise mit organischen Säuren gebildet, wobei dieselben Säuren wie zur Bildung der Additionssalze in Frage kommen, nämlich insbesondere die Essigsäure, aber auch höhere Carbonsäuren, wie z. B. die Propion-, Butter-, Isobutter-, Valerian-, Isovalerian- oder die Pivalinsäure.

Die neuartigen 17β-Alkyl-17α-oxy-estratriene weisen mehrere chirale Zentren auf. Daher gibt es jeweils mehrere stereoisomere Formen jeder Verbindung. Die Verbindungen der Formel I können als Tautomere, Stereoisomere oder geometrische Isomere vorliegen. Die Erfindung umfasst ferner auch alle möglichen Isomeren wie E- und Z-Isomere, S- und R-Enantiomere, Diastereomere, Racemate und Gemische derselben einschließlich der tautomeren Verbindungen. Alle diese isomeren Verbindungen sind - auch wenn jeweils nicht ausdrücklich angegeben - Bestandteil der vorliegenden Erfindung. Die Isomerengemische können nach üblichen Methoden, wie beispielsweise Kristallisation, Chromatographie oder Salzbildung, in die Enantiomeren bzw. E/Z-Isomeren aufgetrennt werden.

Besonders geeignete Verbindungen im erfindungsgemässen Sinne sind Estratriene mit der allgemeinen Formel I, nämlich
- 11 β-Fluor-17β-methyl-7-[5-[methyl(8,8,9,9,9-pentafluornonyl)amino]pentyl]-estra-1,3,5(10)-trien-3,17α-diol
- 11β-Fluor-17β-methyl-7-[5-[(2R)-2-(7,7,8,8,8-pentafluoroctyl)-1-pyrrolidinyl]pentyl]-estra-1,3,5(10)-trien-3,17α-diol
- 11β-Fluor-17β-methyl-7-[5-[(2R)-2-(6,6,7,7,7-pentafluorheptyl)-1-pyrrolidinyl]pentyl]-estra-1,3,5(10)-trien-3,17α-diol
- 4-Chlor-11β-fluor-17β-methyl-7-[5-[methyl(8,8,9,9,9-pentafluornonyl)amino]pentyl]-estra-1,3,5(10)-trien-3,17α-diol
- 4-Brom-11β-fluor-17β-methyl-7-[5-[methyl(8,8,9,9,9-pentafluornonyl)amino]pentyl]-estra-1,3,5(10)-trien-3,17α-diol
- 4-Brom-111β-fluor-17β-methyl-7-[5-[(2R)-2-(7,7,8,8,8-pentafluoroctyl)-1-pyrrolidinyl]pentyl]-estra-1,3,5(10)-trien-3,17α-diol
- 4-Chlor-11β-fluor-17β-methyl-7-[5-[(2R)-2-(7,7,8,8,8-pentafluoroctyl)-1-pyrrolidinyl]pentyl]-estra-1,3,5(10)-trien-3,17α-diol
- 4-Brom-11β-fluor-17β-methyl-7-[5-[(2R)-2-(6,6,7,7,7-pentafluorheptyl)-1-pyrrolidinyl]pentyl]-estra-1,3,5(10)-trien-3,17α-diol
- 4-Chlor-11β-fluoro-17β-methyl-7-[5-[(2R)-2-(6,6,7,7,7-pentafluorheptyl)-1-pyrrolidinyl]pentyl]-estra-1,3,5(10)-trien-3,17α-diol
- 2,4-Dichlor-11β-fluor-17β-methyl-7-[5-[(2R)-2-(7,7,8,8,8-pentafluoroctyl)-1-pyrrolidinyl]pentyl]-estra-1,3,5(10)-trien-3,17α-diol

Die erfindungsgemässen 17β-Alkyl-17α-oxy-estratriene unterscheiden sich von bekannten Verbindungen auch dadurch, dass in 11α-Stellung ein Halogenatom und/oder in 17β-Stellung ein Alkylrest gebunden sind/ist.

Im Gegensatz zu 3,17α-Dihydroxy-estratrienen, die in 17β-Stellung unsubstituiert sind, bilden sich aus den erfindungsgemässen 17β-Alkyl-17α-oxy-estratrienen praktisch keine Metaboliten. Metaboliten können ebenfalls biologisch aktiv sein. Es hat sich nämlich herausgestellt, dass die Estratrienderivate, die durch Oxidation der in 17α-Stellung gebundenen Hydroxygruppe entstehen, wobei ein 17-Oxoderivat entsteht, sehr starke biologische Aktivität aufweisen.

Durch Blockierung der 17β-Stellung durch einen Alkylrest, insbesondere durch eine C₁-C₄-Alkylgruppe, wird diese Oxidationsreaktion unterbunden, so dass auch eine metabolische Vielfalt unterdrückt wird. Die als Wirkstoffe verwendeten erfindungsgemässen Estratriene weisen daher eine speziesunabhängige Wirksamkeit und Aktivität auf. Der Vorteil dieser Verbindungen besteht daher darin, dass die volle Wirksamkeit des Wirkstoffes in einer einzigen Verbindung realisiert wird.

Aus diesem Grunde ergeben sich Vorteile bei der Entwicklung von Arzneimitteln, denn mangels Bildung biologisch aktiver Metaboliten kann die Wirksamkeit bestimmten Strukturprinzipien einfacher zugeordnet werden, so dass eine zielgerichtete Wirkstoffsuche ermöglicht wird.

Ausserdem inhibieren die erfindungsgemässen 17β-Alkyl-17α-oxy-estratriene die Wirkung von Estradiol zu annähernd 100 %. Sie stellen daher Antiestrogene dar.
Zur Untersuchung der Wirksamkeit der erfindungsgemässen Verbindungen wurden in-vivo-Tests an der infantilen Ratte durchgeführt. Hierzu wurde das Uteruswachstum bei peroraler Gabe (p.o.) des Arzneimittels durchgeführt (Test auf antiestrogene Wirkung).

Das Prinzip dieser Methode besteht darin zu untersuchen, welchen Einfluss die Gabe von antiestrogen-wirkenden Verbindungen bei gleichzeitiger Applikation von Estrogenen hat. Bei Nagern reagiert der Uterus auf die Darreichung von Estrogenen nämlich mit einer Gewichtszunahme (sowohl durch Proliferation als auch durch Wassereinlagerung). Dieses Wachstum ist durch gleichzeitige Gabe antiestrogen-wirkender Verbindungen dosisabhängig zu hemmen.

Für die Tests wurden infantile weibliche Ratten mit einem Gewicht von 35 - 45 g bei Versuchsbeginn untersucht. Pro Dosis wurden fünf bis sechs Tiere getestet. Für die p.o. Applikation wurden die Substanzen in einem Teil Ethanol (E) gelöst und mit neun Teilen Erdnussöl (EÖ) aufgefüllt. Zur Eingewöhnung wurden die gerade von den Müttern abgesetzten jungen Ratten einen Tag vor Behandlungsbeginn geliefert und sofort mit Futter - auch in dem Tierkäfig - versorgt. Die Tiere wurden dann täglich einmal drei Tage lang in Kombination mit 0,5 µg Estradiolbenzoat (EB) behandelt. EB wurde immer subcutan (s.c.) appliziert, während die Testsubstanz p.o. verabreicht wurde. 24 Stunden nach der letzten Applikation wurden die Tiere gewogen, getötet und die Uteri entnommen. Von den präparierten Uteri wurden die Feuchtgewichte (ohne Inhalt) ermittelt. Es wurden folgende Kontrolluntersuchungen durchgeführt: Für eine negative Kontrolle wurden 0,2 ml einer E/EÖ-Mischung pro Tier undTag gegeben. Für eine positive Kontrolluntersuchungen wurden 0,5 µg EB/0,1 ml pro Tier und Tag appliziert.

Von den relativen Organgewichten (mg/100 g Körpergewicht) wurden für jede Gruppe die Mittelwerte mit Standardabweichung (X ± SD) sowie die Signifikanz der Unterschiede zur Kontrollgruppe (EB) im Dunnett-Test (p < 0,05) ermittelt. Die Hemmung (in %) gegenüber der EB-Kontrolle wurde mit einem Rechenprogramm ermittelt. Die relative Wirksamkeit der Prüfsubstanzen wurde durch eine Kovarianz- und Regressionsanalyse berechnet.

Untersuchungsergebnisse für ausgewählte Verbindungen sind in Tabelle 1 wiedergegeben. Dort sind Versuchsergebnisse für das Uteruswachstum an der Ratte bei gleichzeitiger Gabe von 0,5 µg EB/0,1 ml s.c. sowie peroraler Darreichung der antiestrogen wirkenden Verbindungen in einer Menge im Bereich von 0,03 mg/kg Körpergewicht wiedergegeben.

**Tabelle 1**

| Verbindung [Bsp.] | Hemmung [%] |
|---|---|
| 1 | 42-59 |
| 2 | 70 |
| 3 | 40 |

Aus Tabelle 1 ist erkennbar, dass die antiestrogene Wirkung bei 50 % liegt, wenn eine Dosierung von etwa 0,03 mg/kg bei peroraler Verabreichung gegeben wurde.

Die erfindungsgemässen Verbindungen sind ebenso wirksam wie oder sogar noch wirksamer als die entsprechenden Verbindungen, die in 17β-Stellung nicht substituiert sind. Gegenüber den in 17β-Stellung nicht substituierten Verbindungen weisen die erfindungsgemässen Estratriene ausserdem eine bessere Verträglichkeit auf, so dass letztere vorzuziehen sind. Die bessere Verträglichkeit ist insbesondere darauf zurückzuführen, dass eine Bildung von Metaboliten weitgehend eingeschränkt ist.

Die erfindungsgemässen Verbindungen zeichnen sich ferner durch eine ausserordentlich hohe Bioverfügbarkeit aus, so dass durch die Applikation der erfindungsgemässen Verbindungen beim betroffenen Patienten hohe Plasmaspiegel erreicht werden können. In Zusammenhang mit der schon erwähnten hohen Verträglichkeit lässt sich so eine erfolgreiche und sichere Therapie durchführen, weil es mit den erfindungsgemässen Verbindungen gelingt, einen Serumspiegel der wirksamen Verbindung einzustellen, der einen ausreichenden Abstand zum Wirkspiegel der entsprechenden Verbindung aufweist. Mit Wirkspiegel ist die Plasmakonzentration des Wirkstoffes gemeint, die zur Erreichung des gewünschten Effektes in der jeweiligen Indikation mindestens erforderlich ist.

Die erfindungsgemässen 17β-Alkyl-17α-oxy-estratriene mit der allgemeinen Formel I sind insbesondere zur Herstellung von Arzneimitteln geeignet. Die Erfindung betrifft daher ausserdem pharmazeutische Präparate, die neben mindestens einem 17β-Alkyl-17α-oxyestratrien mit der allgemeinen Formel I, mindestens einen pharmazeutisch verträglichen Träger enthält.
Die pharmazeutischen Präparate bzw. Zusammensetzungen gemäß der Erfindung werden mit üblichen festen oder flüssigen Trägerstoffen oder Verdünnungsmitteln und üblichen pharmazeutischen und technischen Hilfsstoffen entsprechend der gewünschten Applikationsart mit einer geeigneten Dosierung in an sich bekannter Weise hergestellt. Bevorzugte Zubereitungen bestehen in einer Darreichungsform, die zur oralen, enteralen oder parenteralen, beispielsweise *i.p*. (intraperitonealen), i.v. (intravenösen), *i.m.* (intramuskulären), oder perkutanen, Applikation geeignet ist. Solche Darreichungsformen sind beispielsweise Tabletten, Filmtabletten, Dragees, Pillen, Kapseln, Pulver, Cremes, Salben, Lotionen, Flüssigkeiten, wie Sirupe, Gele, injizierbare Flüssigkeiten, beispielsweise zur *i.p.*, *i.v.*, *i.m.* oder perkutanen Injektion usw. Weiterhin sind auch Depotformen, wie implantierbare Zubereitungen, sowie Suppositorien geeignet. Dabei geben die einzelnen Zubereitungen die erfindungsgemässen Estratriene je nach deren Art allmählich oder die gesamte Menge in kurzer Zeit an den Körper ab.

Zur oralen Verabreichung können Kapseln, Pillen, Tabletten, Dragees und Flüssigkeiten oder andere bekannte orale Darreichungsformen als pharmazeutische Präparate eingesetzt werden. In diesem Falle können die Arzneimittel in der Weise formuliert sein, dass sie die Wirkstoffe entweder in kurzer Zeit freisetzen und an den Körper abgeben oder eine Depotwirkung aufweisen, so dass eine länger anhaltende, langsame Zufuhr von Wirkstoff zum Körper erreicht wird. Die Dosierungseinheiten können neben dem mindestens einen Estratrien einen oder mehrere pharmazeutisch verträgliche Träger enthalten, beispielsweise Stoffe zur Einstellung der Rheologie des Arzneimittels, oberflächenaktive Stoffe, Lösungsvermittler, Mikrokapseln, Mikropartikel, Granulate, Verdünner, Bindemittel, wie Stärke, Zucker, Sorbit und Gelatine, ferner Füllstoffe, wie Kieselsäure und Talkum, Gleitmittel, Farbstoffe, Duftstoffe und andere Stoffe.

Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog zu den Tabletten hergestellten Kernen mit üblicherweise in Dragéeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Die erfindungsgemässen Estratriene können auch in Form einer Lösung formuliert werden, die für die orale Verabreichung bestimmt ist und die neben dem aktiven Estratrien als Bestandteile ein pharmazeutisch verträgliches Öl und/oder eine pharmazeutisch verträgliche lipophile, oberflächenaktive Substanz und/oder eine pharmazeutisch verträgliche hydrophile, oberflächenaktive Substanz und/oder ein pharmazeutisch verträgliches wassermischbares Lösungsmittel enthält.

Um eine bessere Bioverfügbarkeit der erfindungsgemässen Wirkstoffe zu erreichen, können die Verbindungen auch als Cyclodextrinchlatrate formuliert werden. Hierzu werden die Verbindungen mit α-, β- oder γ-Cyclodextrin oder deren Derivaten umgesetzt.

Falls Cremes, Salben, Lotionen und äusserlich anwendbare Flüssigkeiten eingesetzt werden sollen, müssen diese so beschaffen sein, dass die erfindungsgemässen Verbindungen dem Körper in ausreichender Menge zugeführt werden. In diesen Darreichungsformen sind Hilfsstoffe enthalten, beispielsweise Stoffe zur Einstellung der Rheologie der Arzneimittel, oberflächenaktive Mittel, Konservierungsmittel, Lösungsvermittler, Verdünner, Stoffe zur Erhöhung der Permeationsfähigkeit für die erfindungsgemässen Estratriene durch die Haut, Farbstoffe, Duftstoffe und Hautschutzmittel, wie Konditionierer und Feuchteregulatoren. Zusammen mit den erfindungsgemässen Verbindungen können auch andere Wirkstoffe in dem Arzneimittel enthalten sein [Ullmanns Enzyklopädie der technischen Chemie, Band 4 (1953), Seiten 1 - 39; J. Pharm. Sci., 52, 918 ff. (1963); H. v.Czetsch-Lindenwald, Hilfsstoffe für Pharmazie und angrenzende Gebiete; Pharm. Ind., 2, 72 ff (1961); Dr. H. P. Fiedler, Lexikon der Hilfsstoffe für Pharmazie , Kosmetik und angrenzende Gebiete, Cantor AG, Aulendorf/Württ., 1971].

Die erfindungsgemässen Substanzen können auch in geeigneten Lösungen, wie beispielsweise physiologischer Kochsalzlösung, als Infusions- oder Injektionslösung zur Anwendung kommen. Für die parenterale Applikation können die Wirkstoffe in einem physiologisch verträglichen Verdünnungsmittel gelöst oder suspendiert sein. Als Verdünnungsmittel sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Zur Formulierung eines injizierbaren Präparats kann ein beliebiger flüssiger Träger verwendet werden, in dem die erfindungsgemässen Verbindungen gelöst oder emulgiert sind. Diese Flüssigkeiten enthalten häufig auch Stoffe zur Regulation der Viskosität, oberflächenaktive Stoffe, Konservierungsstoffe, Lösungsvermittler, Verdünner und weitere Zusatzstoffe, mit denen die Lösung isotonisch eingestellt wird. Zusammen mit den Estratrienen können auch andere Wirkstoffe verabreicht werden.

Die erfindungsgemässen Estratriene können auch in Form einer Depotinjektion oder eines Implantatpräparats, beispielsweise subkutan, angewendet werden. Derartige Präparate können so formuliert sein, dass eine verzögerte Wirkstoff-Freigabe ermöglicht wird. Hierzu können bekannte Techniken eingesetzt werden, beispielsweise sich auflösende oder mit einer Membran arbeitende Depots. Implantate können als inerte Materialien beispielsweise biologisch abbaubare Polymere oder synthetische Silikone, beispielsweise Silikonkautschuk, enthalten. Die Estratriene können ferner zur perkutanen Verabreichung beispielsweise in ein Pflaster eingearbeitet werden.

Es ist auch möglich, die erfindungsgemässen Substanzen in ein transdermales System einzuarbeiten und sie damit transdermal zu applizieren.
Um einen verbesserten transdermalen Hautfluß zu erreichen, der therapeutisch wirksame Blutspiegel erzeugt, können die erfindungsgemässen Verbindungen auch analog wie es für andere Antiestrogene in der WO 01/76608 beschrieben ist, in Transdermalsysteme eingearbeitet werden. Diese Transdermalsysteme zeichnen sich durch ein spezielles Verhältnis von 2 Penetrationsverstärkern, insbesondere Laurinsäue und Propylenglycol, aus.

Die Dosierung der erfindungsgemässen Substanzen der allgemeinen Formel I wird vom behandelnden Arzt bestimmt und hängt unter anderem von der verabreichten Substanz, dem Verabreichungsweg, der zu behandelnden Erkrankung und von der Schwere der Erkrankung ab. Die zu verabreichende Menge der Verbindungen schwankt innerhalb eines weiten Bereichs und kann jede wirksame Menge abdecken. In Abhängigkeit vom zu behandelnden Zustand und von der Art der Verabreichung kann die Menge der verabreichten Verbindung 0.02 - 20 mg/kg Körpergewicht, vorzugsweise 0.1 - 1 mg/kg Körpergewicht, je Tag betragen. Beim Menschen entspricht dies einer täglichen Dosis von 1 - 1000 mg. Die bevorzugte tägliche Dosierung beim Menschen beträgt 5 - 50 mg. Dies gilt insbesondere für die Tumortherapie. Die Dosis kann als einmal zu verabreichende Einzeldosis oder unterteilt in zwei oder mehrere Tagesdosen gegeben werden.

Die Verbindungen der allgemeinen Formel I stellen Verbindungen mit sehr starker antiestrogener Wirksamkeit dar.
Die Verbindungen eignen sich zur Therapie von estrogen-abhängigen Erkrankungen, zum Beispiel Mammacarcinom (second-line Therapie des Tamoxifen-resistenten Mammacarcinoms; zur adjuvanten Behandlung des Mammacarcinoms anstelle von Tamoxifen), Endometriumcarcinom, Ovarialkarzinom, Prostatahyperplasie, anovulatorische Infertilität und Melanom.

Die Verbindungen der allgemeinen Formel I können gemeinsam mit Antigestagenen (kompetitiven Progesteronantagonisten) zur Behandlung hormonabhängiger Tumoren verwendet werden (EP 310 542 A).

Die Verbindungen der allgemeinen Formel I können auch gemeinsam mit anderen antiproliferativ wirksamen Wirkstoffen kombiniert werden. Bevorzugt ist die Kombination mit Aromatasehemmern wie Anastrozol, Letrozol oder Exemestan.

Weitere Indikationen, in denen die Verbindungen der allgemeinen Formel I zum Einsatz kommen können, ist der männliche Haarausfall, eine diffuse Alopecie, eine durch eine Chemotherapie hervorgerufene Alopecie sowie Hirsutismus (Hye-Sun Oh und Robert C. Smart, Proc. Natl. Acad. Sci. USA, 93 (1996) 12525 - 12530).
Außerdem können die Verbindungen der allgemeinen Formel I zur Herstellung von Medikamenten zur Behandlung von gynäkologischen Erkrankungen wie beispielsweise der Endometriose verwendet werden.
Für gynäkologische Erkrankungen können die erfindungsgemäßen Verbindungen auch kombiniert werden mit Gestagen und/oder Estrogenen. Die Verbindungen der allgemeinen Formel I können beispielsweise als Komponente in den u. a. in der EP 346 014 B1 beschriebenen Produkten verwendet werden, die ein Estrogen und ein reines Antiestrogen enthalten, und zwar zur gleichzeitigen, sequentiellen oder getrennten Verwendung für die selektive Estrogentherapie peri- oder postmenopausaler Frauen.

Ferner kann man die Verbindungen der allgemeinen Formel I zur Herstellung pharmazeutischer Zusammensetzungen für die männliche und weibliche Fertilitätskontrolle einsetzen (männliche Fertilitätskontrolle: DE 195 10 862.0 A).

Die erfindungsgemässen Estratriene können analog zu bekannten Verfahren hergestellt werden:

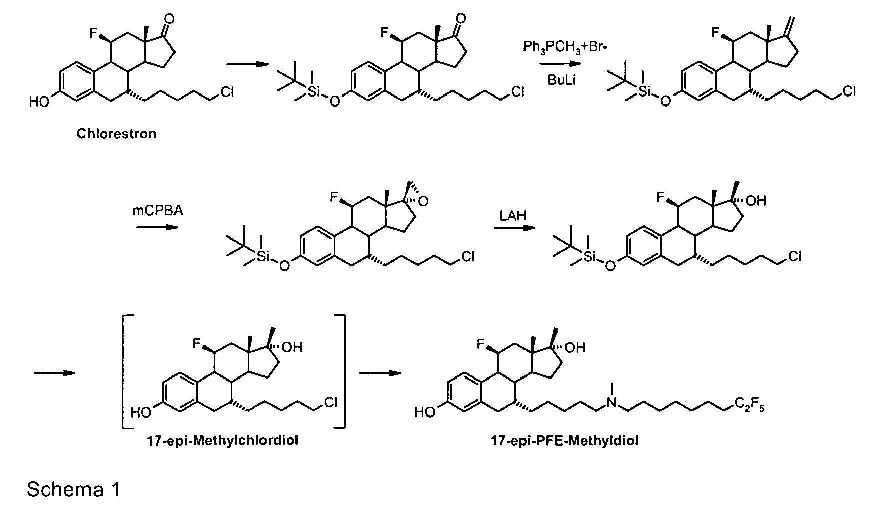

In Schema 1 ist ein Reaktionsschema wiedergegeben, nach dem die erfindungsgemässen Verbindungen hergestellt werden können.

Grundsätzlich können alle aufgeführten Verbindungen, ausgehend von der 17-Oxoverbindung, hergestellt werden. Die Herstellung der 17-Oxoverbindungen ist beispielsweise in WO 99/33855 exemplarisch [CAS: 204138-84-1 (Cl-on, 2b), CAS: 204138-92-1 (Br-on, 1g), CAS: 204138-93-2(Br-ol) beschrieben. Andere Derivate als die ausdrücklich in diesem Dokument offenbarten Verbindungen mit demselben Substitutionsmuster können analog hergestellt werden. In gleicher Weise können die erfindungsgemässen Estratriene auch, ausgehend von den 17α-Hydroxy- oder 17α-Alkoxyverbindungen ("17α-OH"), hergestellt werden. Die Herstellung dieser Derivate ist ebenfalls beispielsweise in WO 99/33855 angegeben. In gleicher Weise ist in diesem Dokument auch die Herstellung der 17α-Hydroxy- oder 17α-Alkoxyverbindungen sowie der 17-Oxoverbindungen mit Amingruppierung in der Seitenkette in 7α-Stellung offenbart. Soweit die Herstellung der Ausgangsverbindungen nicht beschrieben wird, sind die Ausgangsverbindungen bekannt und käuflich, oder die Verbindungen werden analog zu den beschriebenen Verfahren synthetisiert. Nachfolgend wird die Herstellung einiger Vorstufen, Zwischenprodukte und Produkte exemplarisch beschrieben.

Bei der Herstellung der erfindungsgemässen Substanzen bedient man sich beispielsweise folgender Verfahren (siehe hierzu auch EP 0138 504 B1; WO 97/45441 A1; WO 98/07740 A1; WO 99/33855 A1):

Die Seitenkette in 7α-Stellung kann beispielsweise nach der in WO 98/07740 A1 angegebenen Vorgehensweise aufgebaut werden.

In den nachfogend beschriebenen Verfahrensschritten haben die Reste R¹, R². R³, R⁴, R⁵, R⁶, Hal, R^{17"}, R^{17"}, U, V, X, Y, E die oben angegebenen Bedeutungen.
PG steht für eine Schutzgruppe z.B. Ether, Ester oder Carbonate. Insbesondere sind Alkyl- oder Silylether geeignet, wobei Butyldimethylsilyl- und Tetrahydropyranylether bevorzugt sind.
LG steht für eine Fluchtgruppe z.B. eine Sulfonatgruppe oder Halogen. Insbesondere geeignet sind Tosylat- oder Mesylatgruppen, sowie Chlor, Brom oder Iod. Bevorzugt sind Chlor oder eine Mesylatgruppe.
R²⁰ steht für Wasserstoff oder einen gegebenenfalls ein- oder mehrfach fluorierten C₁-C₃-Alkyl-, C₂-C₃-Alkenyl- oder C₂-C₃-Alkinylrest.

Im optionalen Verfahrensschritt a) wird die 3-Hydroxygruppe eines 11-Halogen-7-kettensubstituierten 3-hydroxyestr-17-ons der allgemeinen Formel **III** (WO 99/33855) durch Umsetzung mit einem Silylierungsmittel oder Dihydropyran zur z.B. als Silylether bzw. Tetrahydro-pyranylether geschützten Verbindung der allgemeinen Formel **IV** umgesetzt. Dies wird in einem aprotischen Lösemittel z.B. Dichlormethan in Gegenwart einer Base z.B. Imidazol bzw. einer Säure z.B. Pyridinium-para-toluolsulfonsäure bei Raumteperatur (0 - 100 °C) über 3 Stunden (1 bis 24 Stunden) hergestellt. 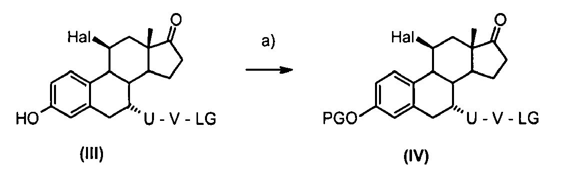 Oder Verfahrensschritt a) wird gemäß (D.W. Hansen und D. Pilipauskas J. Org. Chem. (1985) 945 oder K.F. Bernady et al. J. Org. Chem. (1979) 1438) durchgeführt.

Das geschützte oder freie 7α-Substituierte Estronderivat der allgemeinen Formel (IV) kann mittels einer Wittig-Reaktion z.B. mit einem Methyltriphenylphosphoniumhalogenid in einem aprotischen Lösemittel z.B. Tetrahydrofuran, Dimethylsulfoxid oder Toluol in Gegenwart einer Base z.B. Butyllithium bei Raumtemperatur (20 - 100 °C) über 8 Stunden (1 bis 24 Stunden) ins 17-Exomethylensteroid der allgemeinen Formel (V) überführt werden (Verfahrensschritt b)).

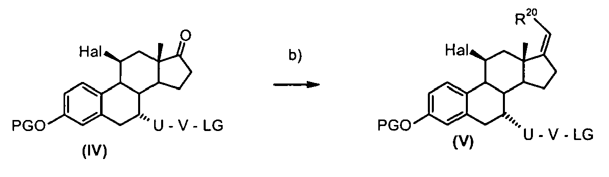

Das exocyclische Olefin der allgemeinen Formel (V) kann im Schritt c) z.B. mit *meta-*Chlorperbenzoesäure in einem aprotischen Lösemittel z.B. Dichlormethan bei Raumtemperatur (0 - 100 °C) über 4 Stunden (1 - 18 Stunden) ins 17-Spiroepoxid der allgemeinen Formel (VI) überführt werden. Das Rohprodukt (VI) dieser Oxidation ist nicht lagerstabil und kann umgehend ohne Aufreinigung im Schritt d) z.B. mit Lithiumaluminiumhydrid in einem aprotischen Lösemittel z.B. Tetrahydrofuran oder Toluol bei 10 °C (0 - 80 °C) über 2 Stunden (1 - 24 Stunden) ins 17β-Alkyl-estra-3,17α-diol der allgemeinen Formel (VII) überführt werden, wobei die Gruppe -CH₂-R²⁰ in Position 17 den Rest R^{17"} darstellt.

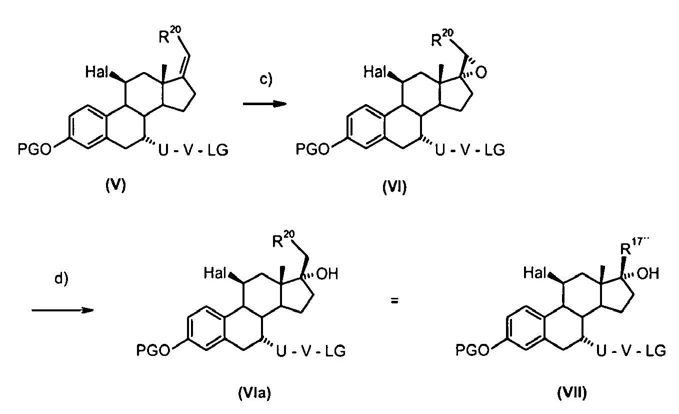

Das geschützte oder freie 17β-Alkyl-estra-3,17α-diol der allgemeinen Formel (VII) kann optional im Schritt e) z.B. durch Zugabe eines Halogenierungsmittels z.B. 2,3,4,5,6,6-Hexachlor-2,4-cyclohexadien-1-on in einem polaren, aprotischen Lösemittel z.B. Dimethylformamid bei Raumtemperatur (0 - 100 °C) über 22 Stunden (4 - 48 Stunden) ins halogenierte Estradiol der allgemeinen Formel (VIII) überführt werden.

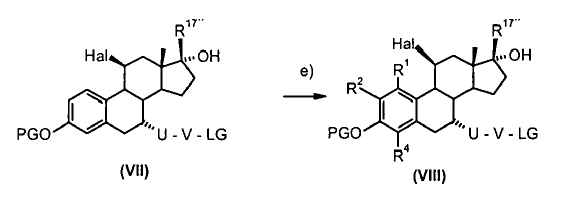

Das geschützte oder freie halogenierte Estradiol der allgemeinen Formel (VIII) kann optional im Schritt f) z.B. durch Zugabe eines Acylierungs- der Veretherungsmittels z.B. Acylanhydrid, Acyl- oder Alkylhalogenid in einem polaren, aprotischen Lösemittel z.B. Pyridin oder Dimethylformamid bei Raumtemperatur (0 - 100 °C) über 12 Stunden (4 - 48 Stunden) vor der eventuellen Schutzgruppenabspaltung in 3-Stellung mit einer organischen Säure z.B. Oxalsäure oder Fluorid z.B. Tetrabutylammoniumfluorid in einem polaren Lösemittel z.B. Wasser oder Ethanol bei Raumteperatur (0 - 100 °C) über 12 Stunden (4 - 48 Stunden) in ein Estradiolderivat der allgemeinen Formel (IX) überführt werden.

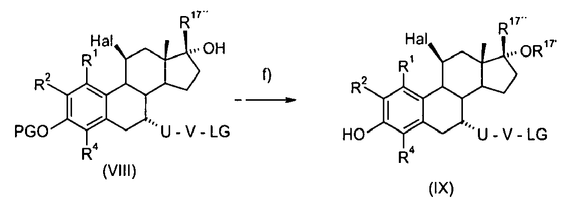

Das Estradiolderivat der allgemeinen Formel (IX) kann in einem Schritt g) mit einem Aminbaustein der allgemeine Formel (XV) in einem polaren, aprotischen Lösemittel z.B. Dimethylformamid ggf. in Gegenwart einer anorganischen Base z.B. Natriumcarbonat sowie einem Iodidsalz z.B. Natriumiodid bei Raumtemperatur (20 - 100 °C) über 8 Stunden (4 - 48 Stunden) ins Amin der allgemeinen Formel (I) überführt werden.

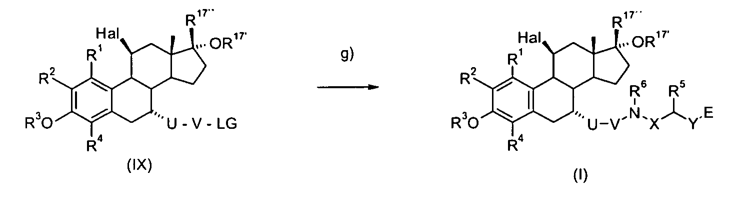

### Allgemeine Herstellung eines Aminbausteins der allgemeinen Formel (XV):

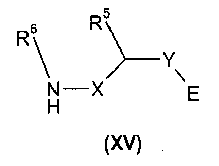

lodierung:

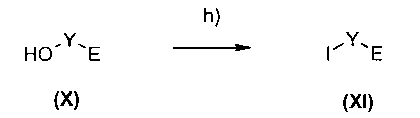

Ein Alkohol der allgemeinen Formel (X) kann in einem Schritt h) z.B. durch Eintropfen in eine Reaktionslösung aus Triphenylphosphin und Iod in Dichlormethan bei Raumtemperatur (20 - 100 °C) über 1 Stunde (0.25 - 8 Stunden) ins Iodid der allgemeinen Formel (XI) überführt werden.

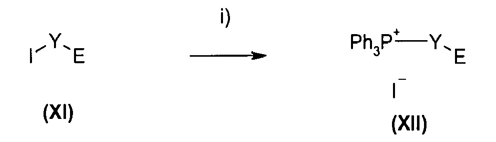

Durch Zugabe des Iodids der allgemeinen Formel (XI) zu einer Suspension von Triphenylphosphin in aprotischen polaren Lösemittel z.B. Acetonitril und Rühren bei Siedetemperatur (40 - 100 °C) über 8 Stunden (2 - 24 Stunden) kann in einem Schritt i) das Wittigsalz der allgemeinen Formel (XII) erhalten werden.

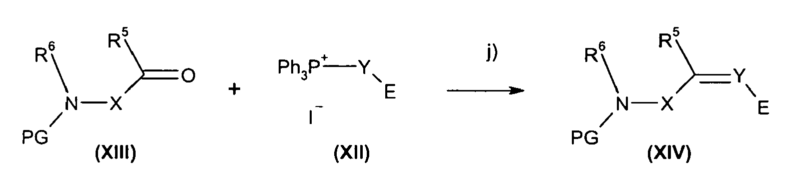

In einem Schritt j) kann ein Aldehyd b.z.w. Keton der allgemeinen Formel (XIII) bei -40°C (-78 - 30°C) zu einer Reaktionslösung eines Wittigsalzes der allgemeinen Formel (XII) und einer Base z.B. Natriumhexamethyldisilazan in einem aprotischen Lösemittel z.B. Tetrahydrofuran zugetropft, auf Raumtemperatur (0 - 80 °C) erwärmt und durch eine weitere Stunde (0.5 - 10 Stunden) rühren bei dieser Temperatur in ein gegebenfalls geschütztes Olefin der allgemeinen Formel (XIV) überführt werden.

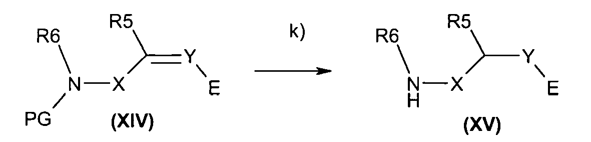

Ein gegebenenfalls geschütztes Olefin der allgemeinen Formel (XIV) kann in einem Schritt k) in einem polaren Lösemittel z.B. wässrigem Ethanol mit Wasserstoff unter Normaldruck (1 - 20 bar) in Gegenwart eines Hydrierkatalysators z.B. Platinoxid bei Raumtemperatur (0 - 80 °C) in 1 Stunde (0.5 - 8 Stunden) hydriert und gegebenenfalls zum Amin der allgemeinen Formel (XV) z.B. mit einer Säure z.B. Salzsäure entschützt werden.

### Herstellung eines Alkohols der allgemeinen Formel (X):

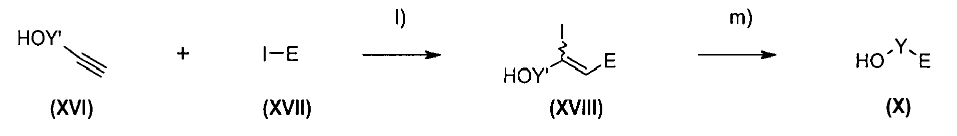

Ein Alkinol der allgemeinen Formel (XVI) (wobei -Y'(CH₂)₂- für -Y- steht) kann z.B. in einem Schritt I) in einem polaren aprotischen Lösemittel z.B. Acetonitril in Gegenwart von Natriumhydrogencarbonat und Natriumdithionit bei -10 °C (-30 - 30 °C) mit einem Iodid der allgemeinen Formel (XVII) ins Vinyliodid der allgemeinen Formel (XVIII) überführt werden. Das Vinyliodid der allgemeinen Formel (XVIII) kann in einem Schritt m) mit Wasserstoff unter Normaldruck (1 -20 bar) in Gegenwart eines Hydrierkatalysators z.B. Palladium auf Aktivkohle bei Raumtemperatur (0 - 80 °C) in 1 Stunde (0.5 - 8 Stunden) zum Alkohol der allgemeinen Formel (X) hydriert werden.

### A. Herstellung der erfindungsgemäßen Verbindungen

### Beispiel 1

### [5-(11β-Fluor-3,17α-dihydroxy-17β-methylestra-1,3,5(10)-trien-7α-yl)pentyl](methyl)(8,8,9,9,9-pentafluornonyl)amin

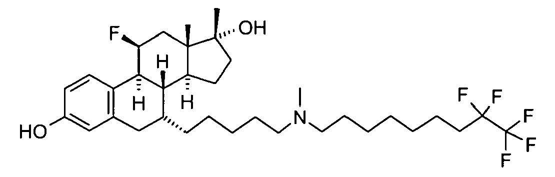

### 1.1 Herstellung der Intermediate

### 1.1.1 3-(tert-Butyldimethylsilyloxy)-7α-(5-chlorpentyl)-11β-fluor-estra-1,3,5(10)-trien-17-on

Eine Lösung von 15.7 g 7α-(5-Chlorpentyl)-11β-fluor-3-hydroxy-17β-methyl-estra-1,3,5(10)-trien-17-on [WO 99/33855, CAS: 204138-84-1] in 160 ml Dichlormethan wurde bei Raumtemperatur mit 3.81 g Imidazol versetzt, auf 10 °C gekühlt, portionsweise mit 8.44 g *tert*-Butyldimethylsilylchlorid versetzt und 3 Stunden bei Raumtemperatur gerührt. Zur Aufarbeitung wurden unter Eiskühlung eine gesättigte Natriumhydrogencarbonatlösung (40 ml) sowie 80 ml Wasser zugegeben, die Phasen getrennt, die organische Phase über Natriumsulfat getrocknet und im Vakuum eingeengt. Man erhielt 21 g rohes 3-(*tert-*Butyldimethylsilyloxy)-7α-(5-chlorpentyl)-11 β-fluor-estra-1,3,5(10)-trien-17-on.
H-NMR: 400 MHz, CDCl3, δ = 7.16 (d, 1H), 6.67 (d(br), 1H), 6.58 (s(br), 1H), 5.58 (d, 1H), 3.51 (t, 2H), 0.98 (s, 9H), 0.91 (s, 3H), 0.19 (s, 6H).

### 1.1.2 3-(tert-Butyldimethylsilyloxy)-7α-(5-chlorpentyl)-11β-fluor-17-methylen-estra-1,3,5(10)-trien

Ein Lösung von 28.58 g Methyltriphenylphosphoniumbromide in 500 ml Tetrahydrofuran wurde bei Raumtemperatur über 10 Minuten mit 36 ml einer Butyllithiumlösung (2.5 M in Hexan) versetzt, 20 Minuten bei Raumtemperatur gerührt, auf 70 °C erwärmt, tropfenweise über 80 Minuten mit einer Lösung von 10.14 g 3-(*tert*-Butyldimethylsilyloxy)-7α-(5-chlorpentyl)-11β-fluor-estra-1,3,5(10)-trien-17-on in 280 ml Tetrahydrofuran versetzt und 2 Stunden bei 95 °C Badtemperatur gerührt. Zur Aufarbeitung wurden unter Eiskühlung 160 ml Wasser zugetropft, die Phasen getrennt, mit Ethylacetat extrahiert, die vereinigten organischen Phasen mit gesättigter Kochsalzlösung gewaschen, über Natriumsulfat getrocknet und im Vakuum eingeengt.

Man erhielt 27 g erstes Rohprodukt. Dieses wurde 4mal bei 40 °C mit 100 ml Hexan gerührt, dekantiert, im Vakuum getrocknet. Man erhielt 11.6 g gereinigtes Rohprodukt 3-(*tert-*Butyldimethylsilyloxy)-7α-(5-chlorpentyl)-11 β-fluor-17-methylen-estra-1,3,5(10)-trien.
H-NMR: 300 MHz, CDCl3, δ = 7.18 (d, 1H), 6.67 (m, 1H), 6.57 (s(br), 1H), 5.57 (d(br), 1H), 4,67 (m, 2H), 3.51 (t, 2H), 0.97 (s, 9H), 0.19 (s, 6H).

### 1.1.3 3-(tert-Butyldimethylsilyloxy)-7α-(5-chlorpentyl)-11β-fluor-(17R)-spiro-[estra-1,3,5(10)-trien-17,2'-oxiran]

Ein Lösung von 5.32 g 3-(*tert*-Butyldimethylsilyloxy)-7α-(5-chlorpentyl)-11β-fluor-17-methylen-estra-1,3,5(10)-trien in 55 ml Dichlormethan wurde bei Raumtemperatur portionsweise mit 3.05 g m-Chlorperbenzoesäure versetzt und 4 Stunden gerührt. Zur Aufarbeitung wurde mit 50 ml gesättigter Nartriumhydrogencarbonatlösung sowie 50 ml Methyl-*tert*-butylether versetzt, die Phasen getrennt, mit Methyl-*tert*-butylether extrahiert, die vereinigten organischen Phasen über Natriumsulfat getrocknet und im Vakuum eingeengt. Man erhielt 5.6 g rohes 3-(*tert*-Butyldimethylsilyloxy)-7α-(5-chlorpentyl)-11β-fluor-(17R)-spiro-[estra-1,3,5(10)-trien-17,2'-oxiran]
H-NMR: 300 MHz, CDCl3, δ = 7.16 (d, 1H), 6.67 (m, 1H), 6.57 (s(br), 1H), 5.57 (d, 1H), 3.51 (t, 2H), 3.22 (s, 1H), 1.02 (s(br), 3H), 0.98 (s, 12H), 0.19 (s, 6H).

### 1.1.4 3-(tert-Butyldimethylsilyloxy)-7α-(5-chlorpentyl)-11β-tluor-17β-methyl-estra-1,3,5(10)-trien-17α-ol

Zu einer 2 M-Lösung von Lithiumaluminiumhydrid (5.03 ml) wurde bei 10 °C eine zweite Lösung von 5.27 g 3-(*tert*-Butyldimethylsilyloxy)-7α-(5-chlorpentyl)-11β-fluor-(17R)-*spiro-*[estra-1,3,5(10)-trien-17,2'-oxiran] in 20 ml Terahydrofuran getropt und 2 Stunden bei Raumtemperatur gerührt. Zur Aufarbeitung wurde mit 8 ml Aceton sowie 70 ml Citronensäurelösung versetzt, mit 80 ml Ethylacetat verdünnt, die Phasen getrennt, Wasserphase mit Ethylacetat extrahiert, die vereinigten organischen Phasen mit Kochsalzlösung gewaschen, über Natriumsulfat getrocknet, im Vakuum eingeengt und chromatographiert an Kieselgel mit Hexan / Ethylacetat. Man erhielt 2.75 g reines 3-(*tert-*Butyldimethylsilyloxy)-7α-(5-chlorpentyl)-11β-fluor-17β-methyl-estra-1,3,5(10)-trien-17α-ol.
H-NMR: 300 MHz, CDCl3, δ = 7.17 (d, 1H), 6.65 (m, 1H), 6.57 (s(br), 1H), 5.52 (d, 1H), 3.51 (t, 2H), 1.26 (d, 3H), 0.98 (s, 9H), 0.91 (d, 3H), 0.19 (s, 6H).

### 1.1.5 Methyl-(8,8,9,9,9-pentafluornonyl)-amin

In eine Lösung von 2.9 g 8,8,9,9,9-Pentafluor-nonyltosylat [WO 99/33855, Seite 20, CAS: 228570-38-5] in 10 ml abs. Tetrahydrofuran wurde bei -20°C 4.0 g Methylamin kondensiert und über Nacht im Druckgefäß bei Raumtemperatur gerührt. Nachdem das Druckgefäß bei -20°C geöffnet wurde, ließ man auf Raumtemperatur kommen, um überschüssiges Methylamin abdampfen zu lassen. Die Reaktionslösung wurde in Dichlormethan aufgenommen, mit Wasser gewaschen, über Magnesiumsulfat getrocknet und i. Vak. eingeengt. Es wurden 1.58 g Methyl-(8,8,9,9,9-pentafluornonyl)-amin als Rohprodukt erhalten.
H-NMR: 300 MHz, CDCl3, δ = 2.60 (t, 2H), 2.47 (s, 3H), 1.94-2.14 (m, 2H), 1.57-1.68 (d, 2H), 1.48-1.56 (m, 2H), 1.34-1.46 (m, 6H).

### 1.2 Herstellung des Endproduktes

Eine Lösung von 1.1 g 3-(*tert*-Butyldimethylsilyloxy)-7α-(5-chlorpentyl)-11β-fluor-17β-methyl-estra-1,3,5(10)-trien-17α-ol in 6.29 ml Dimethylformamid wurde mit 0.22 g Natriumcarbonat, 0.63 g Natriumiodid sowie 0.78 g (8,8,9,9,9-Pentafluornonyl)-methyl-amin versetzt und 5 Stunden bei 80 °C gerührt. Zur Aufarbeitung wurde mit 10 ml Ethylacetat verdünnt, mit Wasser, Natriumhydrogencarbonat-Lösung gewaschen, die Phase über Magnesiumsulfat getrocknet, filtriert und im Vakuum eingeengt. Dieses Rohprodukt wurde in 9.44 ml Ethanol gelöst, mit 0.73 g Kaliumfluorid sowie 0.2 ml Wasser versetzt und 14 Stunden bei Raumtemperatur gerührt. Anschließend wurde im Vakuum eingeengt und an Kieselgel mit Hexan / Ethylacetat chromatographiert. Man erhielt 577 mg reines [5-(11β-Fluor-3,17α-dihydroxy-17β-methylestra-1,3,5(10)-trien-7α-yl)pentyl](methyl)(8,8,9,9,9-pentafluornonyl)amin.
H-NMR: 600 MHz, CDCl3, δ = 7.18 (d, 1H), 6.63 (m, 1H), 6.52 (s(br), 1H), 5.60 (d, 1H), 2.21 (s, 3H), 1.26 (s, 3H), 0.88 (d, 3H).

### Beispiel 2

### 11β-Fluor-17β-methyl-7α-[5-[(2R)-2-(7,7,8,8,8-pentafluoroctyl)-1-pyrrolidinyl]pentyl]-estra-1,3,5(10)-trien-3,17α-diol

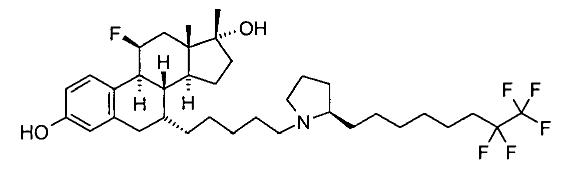

### 2.1 Herstellung der Intermediate

### 2.1.1 (R)-2-(7,7,8,8,8-Pentafluoro-octyl)-pyrrolidin

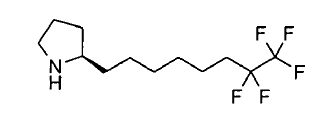

### a) 6,6,7,7,7-Pentafluoro-4-iodo-hept-4-en-1-ol

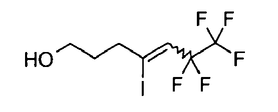

Bei -12°C wurden zu einer Lösung von 150 g 4-Pentin-1-ol in 1.5 I Acetonitril und 1.14 I Wasser 507 g Pentafluoriodethan gegeben. Anschließend wurde eine Mischung aus 150 g Natriumhydrogencarbonat und 294 g Natriumdithionit (85%ig) zugesetzt und für 1 Stunde bei -10 bis 0°C gerührt. Nach DC-Kontrolle wurde das Reaktionsgemisch auf Wasser, extrahiert mit Essigester, mit gesättigter Natriumchloridlösung gewaschen und über Natriumsulfat getrocknet. Nach der Filtration wurde vorsichtig am Rotationsverdampfer bis 100 mbar eingeengt. Man erhielt 6,6,7,7,7-Pentafluoro-4-iodo-hept-4-en-1-ol als Rohrodukt (650 g).

### b) 6,6,7,7,7-Pentafluorheptan-1-ol CAS [344452-11-5]

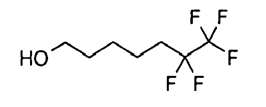

Bei Raumtemperatur wurden zu einer Lösung von 650 g rohem 6,6,7,7,7-Pentafluor-4-iod-hept-4-en-1-ol in 3 l Essigester und 500 ml Triethylamin 118 g Palladium-Kohle gegeben und anschließend bei Normaldruck-Wasserstoffatmosphäre hydriert, wobei der Reaktionsfortschritt per 1H-NMR verfogt wurde. Nach Filtration und Wäsche des Katalysators mit Essigester wurde die organische Phase mit Wasser, 1 %iger Salzsäure sowie gesättigter Natriumchloridlösung gewaschen. Nach dem Trocknen mit Natriumsulfat und vorsichtigem Einengen des Solvents bis 50 mbar wurde im Ölpumpenvakuum (0.5 mbar) bei 65°C zu 6,6,7,7,7-Pentafluoro-heptan-1-ol (235 g) destilliert.
H-NMR: 400 MHz, CDCl₃, δ = 3.66 (t, 2H), 2.03 (m, 2H), 1.40-1.70 (m, 7H).

### c) 1,1,1,2,2-Pentafluoro-7-iodo-heptan

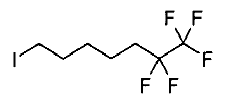

Bei Raumtemperatur wurden unter Kühlung zu einer Lösung von 64.25 g Triphenylphosphin in 500 ml Dichlormethan 61.56 g Iod gegeben und 15 Minuten gerührt. Anschließend wurde über einen Zeitraum von 1 Stunde tropfenweise mit der Lösung von 50 g 6,6,7,7,7-Pentafluoro-heptan-1-ol in 60 ml Dichlormethan versetzt und 1 Stunde nachgerührt. Nach Aufgabe auf Wasser wurde mit Dichlormethan extrahiert, die organische Phase mit gesättigter Natriumchloridlösung gewaschen, über Natriumsulfat getrocknet, abfiltrierte und vorsichtig am Rotationsverdampfer eingeengt. Der Rückstand wurde mit 300 ml Hexan digeriert und die erhaltene Hexanphase bei 10 mbar destilliert, wobei man 1,1,1,2,2-Pentafluoro-7-iodo-heptan (62.4 g) erhielt.
H-NMR: 300 MHz, CDCl₃, δ = 3.20 (t, 2H), 2.03 (m, 2H), 1.86 (m, 2H), 1.43-1.69 (m, 4H).

### d) (6,6,7,7,7-Pentafluoro-heptyl)-triphenyl-phosphoniumiodid

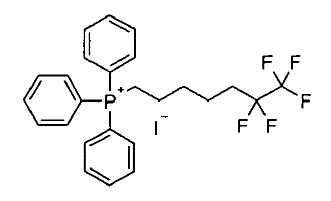

Zu einer Suspension von 260 g Triphenylphosphin in 1.26 l Acetonitril wurden 313 g 1,1,1,2,2-Pentafluoro-7-iodo-heptan gegeben und für 18 Stunden unter Rückfluss erwärmt. Nach Eingen des Solvens wurde 2-mal aus tert-Butylmethylether umkristallisiert. Man erhielt (6,6,7,7,7-Pentafluoro-heptyl)-triphenyl-phosphoniumiodid (567 g) als weißes Pulver.
H-NMR: 300 MHz, CDCl₃, δ = 7.66-7.91 (m, 15 H), 3.76 (m, 2H), 1.50-2.10 (m, 8H).

### e) (S)-2-[(Z)-7,7,8,8,8-Pentafluor-oct-1-enyl]-pyrrolidin-1-carbonsäure-tert-butylester

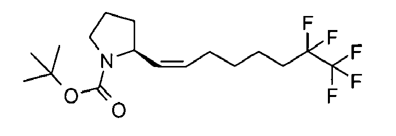

Bei -40°C wurde die Lösung von 468.38 g (6,6,7,7,7-Pentafluorheptyl)-triphenyl-phosphoniumiodid in 3.8 I Tetrahydrofuran innerhalb von 10 Minuten mit 738 ml Natriumhexamethyldisilazan (1M in Tetrahydrofuran) versetzt und 2 Stunden bei dieser Temp. belassen. Anschließend wurde über 15 Minuten die Lösung von 147 g (2S)-2-Formyl-1-pyrrolidinecarbonsäure-tert-butylester, in 850 ml Tetrahydrofuran hinzugegeben, 1 Stunde bei -40°C gerührt, auf Raumtemperatur erwärmt und eine weitere Stunde nachgerührt. Nach der Zugabe von 2.5 L Hexan wurde auf 1/3 eingeengt, mit tert-Butylmethylether versetzt und den ausgefallenen Niederschlag absitzen gelassen. Anschließen wurde filtriert, mit tert-Butylmethylether gewaschen und zur Trockne eingeengt. Flash-Chromatographie an Kieselgel (Hexan / Essigester) lieferte (S)-2-[(Z)-7,7,8,8,8-Pentafluor-oct-1-enyl]-pyrrolidine-1-carbonsäure-tert-butylester (155.7 g).
H-NMR: 300 MHz, CDCl₃, δ = 5.33 (m, 2H), 4.47 (br. s, 1H), 3.37 (m, 2H), 1.41 (s, 9H).

### f) (R)-2-(7,7,8,8,8-Pentafluoroctyl)-pyrrolidin-1-carbonsäure-tert-butylester

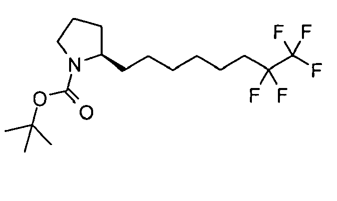

Bei Raumtemp. gab man zu einer Lösung von 61.9 g (S)-2-(7,7,8,8,8-Pentafluoroct-1-enyl)-pyrrolidin-1-carbonsäure-tert-butylester ester in 1.4 l Methanol und 300 ml Wasser 6.19 g Platinoxid und hydrierte unter Normaldruck bis zur vollständigen Wasserstoffaufnahme. Nach der Filtration über eine Schicht Celite wurde eingeengt, in Essigester aufgenommen, die wäßr. Phase abgetrennt, über Natriumsulfat getrocknet und aufkonzentriert. Nach Filtration der Lösung über einen PTFE-Filter engte man zur Trockne ein. Den erhaltenen (R)-2-(7,7,8,8,8-Pentafluoroctyl)-pyrrolidine1- carbonsäure-tert-butylester (59.6 g) wurde als Rohprodukt direkt in die Folgestufe eingesetzt.
H-NMR: 300 MHz, CDCl₃, δ = 3.71 (br. s, 1H), 3.21-3.43 (m, 2H), 1.44 (s, 9H).

### g) (R)-2-(7,7,8,8,8-Pentafluoroctyl)-pyrrolidin

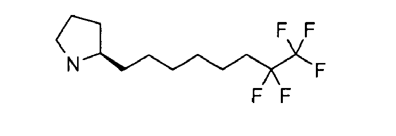

Bei Raumtemp. gab man zu einer Lösung von 35 g (R)-2-(7,7,8,8,8-Pentafluoroctyl)-pyrrolidin-1-carbonsäure-tert-butylester in 636 ml 1,4-Dioxan 35 ml Salzsäure (37%ig) und erhitzte anschließend für 1 Stunde auf 50°C. Anschließend engte man ein, versetzte mit Methylenchlorid, wusch 2 x mit gesättigter Natriumhydrogencarbonatlösung und Wasser, trocknete über Magnesiumsulfat, engte ein und reinigte per Kugelrohrdestillation (Kp 110-125°C, 1 torr). Man erhielt (R)-2-(7,7,8,8,8-Pentafluor-octyl)-pyrrolidin (21.21 g).
H-NMR: 400 MHz, CDCl₃, δ = 3.00 (ddd, 1H), 2.92 (m, 1H), 2.81 (ddd, 1H), 2.00 (m, 2H), 1.87 (m, 1H), 1.52-1.80 (m, 6H), 1.28-1.51 (m, 8H), 1.21 (m, 1H).

### 2.2 Herstellung des Endproduktes

In Analogie zu Schritt 1.2 des Beispiels 1 lieferten 1,1 g 3-(*tert*-Butyldimethylsilyloxy)-7α-(5-chlorpentyl)-11β-fluor-17β-methyl-estra-1,3,5(10)-trien-3,17α-diol mit 860 mg (R)-2-(7,7,8,8,8-Pentafluor-octyl)-pyrrolidin als Amin nach der Flash-Chromatographie 11β-Fluor-17β-methyl-7α-[5-[(2R)-2-(7,7,8,8,8-pentafluoroctyl)-1-pyrrolidinyl]pentyl]-estra-1,3,5(10)-trien-3,17α-diol (772 mg).
H-NMR: 400 MHz, CDCl₃, δ = 7.19 (d, 1H), 6.65 (m, 1H), 6.55 (s(br), 1H), 5.60 (d, 1H), 3.14 (m, 1H), 2.91 (m, 1H), 2.54 (m, 1H), 1.26 (s, 3H), 0.88 (d, 3H).

### Beispiel 3

### 11β-Fluor-17β-methyl-7α-[5-[(2R)-2-(6,6,7,7,7-pentafluorheptyl)-1-pyrrolidinyl]pentyl]-estra-1,3,5(10)-trien-3,17α-diol

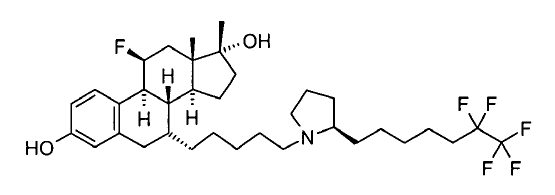

### 3.1 Herstellung der Intermediate

### 3.1.1 (R)-2-(6,6,7,7,7-Pentafluoro-heptyl)-pyrrolidin

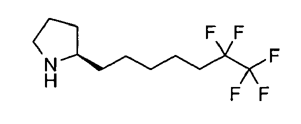

### a) 5,5,6,6,6-Pentafluoro-3-iodo-hex-3-en-1-ol

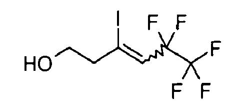

Analog der im Beispiel 2.1.1 a) beschriebenen Methode erhielt man aus 280 g 3-Butin-1-ol und 1136 g Pentafluorethyliodid 5,5,6,6,6-Pentafluoro-3-iodo-hex-3-en-1-ol (1205 g) als Rohprodukt, das direkt in die Folgestufe eingesetzt wurde.

### b) 5,5,6,6,6-Pentafluoro-hexan-1-ol (ZK 6074973, CAS [58556-45-9])

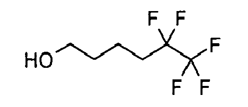

Analog der im Beispiel 2.1.1 b) bespriebenen Methode erhielt man aus 319.5 g 5,5,6,6,6-Pentafluoro-3-iodo-hex-3-en-1-ol 5,5,6,6,6-Pentafluoro-hexan-1-ol (223 g).
H-NMR: 300 MHz, CDCl₃, δ = 3.69 (t, 2H), 2.06 (m, 2H), 1.54-1.77 (m, 5H).

### c) 1,1,1,2,2-Pentafluoro-6-iodo-hexan

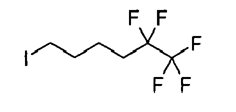

Analog der im Beispiel 2.1.1 c) bespriebenen Methode erhielt man aus 223 g 5,5,6,6,6-Pentafluoro-hexan-1-ol 1,1,1,2,2-Pentafluoro-6-iodo-hexane (223 g).
H-NMR: 300 MHz, CDCl₃, δ = 3.20 (t, 2H), 1.85-2.15 (m, 4H), 1.66-1.79 (m, 2H).

### d) (5,5,6,6,6-Pentafluoro-hexyl)-triphenyl-phosphoniumiodid

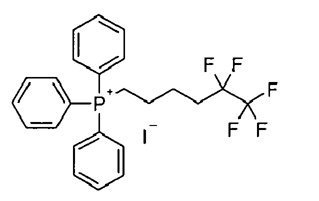

Analog der im Beispiel 2.1.1 d) bespriebenen Methode erhielt man aus 334 g 1,1,1,2,2-Pentafluoro-6-iodo-hexane (5,5,6,6,6-Pentafluoro-hexyl)-triphenyl-phosphoniumiodide (671 g).
H-NMR: 300 MHz, CDCl₃, δ = 7.65-7.92 (m, 15 H), 3.88 (m, 2H), 1.73-2.22 (m, 6H).

### e) (S)-2-[(Z)-6,6,7,7,7-Pentafluor-hept-1-enyl]-pyrrolidin-1-carbonsäure-tert-butylester

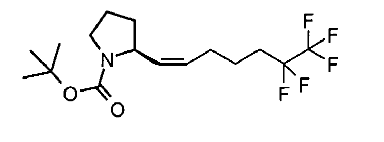

Analog der im Beispiel 2.1.1 e) bespriebenen Methode erhielt man aus 318 g (5,5,6,6,6-Pentafluoro-hexyl)-triphenyl-phosphoniumiodide (671 g) (S)-2-[(Z)-6,6,7,7,7-Pentafluoro-hept-1-enyl]-pyrrolidine-1-carbonsäure-*tert*-butylester (132.7 g).
H-NMR: 300 MHz, CDCl₃, δ = 5.29-5.49 (m, 2H), 4.52 (m, 1H), 3.44 (m, 2H), 2.28 (m, 2H), 1.46 (s, 9H).

### f) (R)-2-(6,6,7,7,7-Pentafluoro-heptyl)-pyrrolidine-1- carbonsäure-tert-butylester

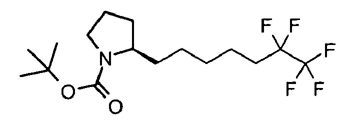

Analog der im Beispiel 2.1.1 f) bespriebenen Methode erhielt man aus 132 g (S)-2-((Z)-6,6,7,7,7-Pentafluoro-hept-1-enyl)-pyrrolidine-1- carbonsäure-tert-butylester den (R)-2-(6,6,7,7,7-Pentafluoro-heptyl)-pyrrolidine-1-carbonsäure-tert-butylester (135 g).
H-NMR: 300 MHz, CDCl₃, δ = 3.72 (br. s, 1H), 3.23-3.39 (m, 2H), 1.44 (s, 9H).

### g) (R)-2-(6,6,7,7,7-Pentafluoro-heptyl)-pyrrolidin

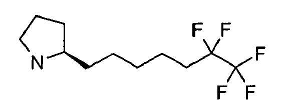

Analog der im Beispiel 2.1.1 g) bespriebenen Methode erhielt man aus 20.5 g (R)-2-(6,6,7,7,7-Pentafluorheptyl)-pyrrolidin-1-carbonsäure-tert-butylester das (R)-2-(6,6,7,7,7-Pentafluoro-heptyl)-pyrrolidin (13 g).
H-NMR: 400 MHz, CDCl₃, δ = 4.09 (br s, 1H), 3.00 (m, 2H), 1.61-1.94 (m, 5H), 1.21-1.59 (m, 9H).

### 3.2 Herstellung des Endproduktes

In Analogie zu Schritt 1.2 des Beispiels 1 lieferten 1,1 g 3-(*tert*-Butyldimethylsilyloxy)-7α-(5-chlorpentyl)-11β-fluor-17β-methyl-estra-1,3,5(10)-trien-3,17α-diol mit 820 mg (R)-2-(7,7,8,8,8-Pentafluor-heptyl)-pyrrolidin als Amin nach der Flash-Chromatographie 11β-Fluor-17β-methyl-7α-[5-[(2R)-2-(6,6,7,7,7-pentafluorheptyl)-1-pyrrolidinyl]pentyl]-estra-1,3,5(10)-trien-3,17α-diol (760 mg).
H-NMR: 400 MHz, CDCl₃, δ = 7.18 (d, 1H), 6.63 (m, 1H), 6.53 (s(br), 1H), 5.60 (d, 1H), 3.16 (m, 1H), 2.90 (m, 1H), 2.54 (m, 1H), 1.26 (s, 3H), 0.88 (d, 3H).

### Beispiel 4

### 4-Chlor-11β-fluor-17β-methyl-7α-[5-[methyl(8,8,9,9,9-pentafluornonyl)amino]pentyl]-estra-1,3,5(10)-trien-3,17α-diol

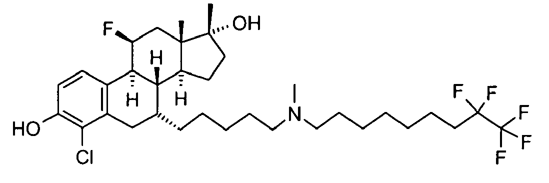

### 4.1 Herstellung der Intermediate

### 4.1.1 3-(tert-Butyldimethylsilyloxy)-4-chlor-7α-(5-chlorpentyl)-11β-fluor-17β-methyl-estra-1,3,5(10)-trien-17α-ol

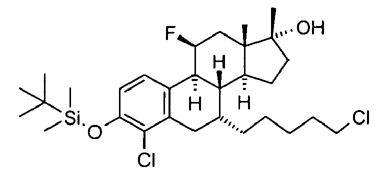

Zu einer Lösung von 732 mg 3-(*tert*-Butyldimethylsilyloxy)-7α-(5-chlorpentyl)-11β-fluor-17β-methyl-estra-1,3,5(10)-trien-17α-ol in 9 ml Dimethylformamid wurde unter Stickstoffatmosphäre 505 mg 2,3,4,5,6,6-Hexachlor-2,4-cyclohexadien-1-on gegeben und 22 Stunden bei Raumtemperatur gerührt. Danach erfolgte eine weitere Zugabe von 126 mg 2,3,4,5,6,6-Hexachlor-2,4-cyclohexadien-1-on, gefolgt von einer letzten 126 mg-Portion nach 7 Stunden. Nachdem weitere 56 Stunden gerührt wurde, wurde das Reaktionsgemisch eingeengt und das Rohprodukt wiederholt an Kieselgel (Hexan / Essigester) chromatographiert. Man erhielt 444 mg 3-(*tert*-Butyldimethylsilyloxy)-4-chlor-7α-(5-chlorpentyl)-11β-fluor-17β-methyl-estra-1,3,5(10)-trien-17α-ol.
H-NMR: 400 MHz, CDCl₃, δ = 7.12 (d, 1H), 6.75 (m, 1H), 5.59 (d, 1H), 3.51 (t, 2H), 3.06 (d, 1H), 1.26 (s, 3H), 1.03 (s, 9H), 0.87 (d, 3H), 0.23 (s, 3H), 0.22 (s, 3H).

### 4.2 Herstellung des Endproduktes

In Analogie zu Schritt 1.2 des Beispiels 1 lieferten 428 mg 3-(*tert*-Butyldimethylsilyloxy)-4-chlor-7α-(5-chlorpentyl)-11β-fluor-17β-methyl-estra-1,3,5(10)-trien-17α-ol mit 284 mg (8,8,9,9,9-Pentafluornonyl)-methyl-amin als Amin nach der Flash-Chromatographie 4-Chlor-11β-fluor-17β-methyl-7α-[5-[methyl(8,8,9,9,9-pentafluornonyl)amino]pentyl]-estra-1,3,5(10)-trien-3,17α-diol (176 mg).
H-NMR: 400 MHz, CDCl₃, δ = 7.20 (d, 1H), 6.89 (m, 1H), 5.59 (d, 1H), 3.00 (d, 1H), 2.18 (s, 3H), 1.26 (s, 3H), 0.87 (d, 3H).

### Beispiel 5

### 4-Brom-11β-fluor-17β-methyl-7α-[5-[methyl(8,8,9,9,9-pentafluornonyl)amino]pentyl]-estra-1,3,5(10)-trien-3,17α-diol

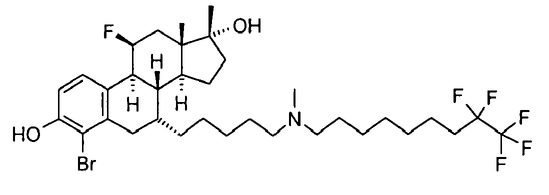

### 5.1 Herstellung der Intermediate

### 5.1.1 7α-(5-Chlorpentyl)-11β-fluor-17β-methyl-estra-1,3,5(10)-trien-3,17α-diol

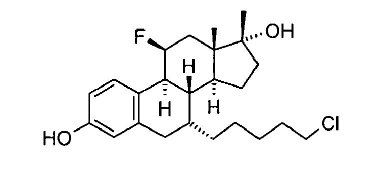

Zu einer Lösung von 3.2 g 3-(*tert*-Butyldimethylsilyloxy)-7α-(5-chlorpentyl)-11β-fluor-17β-methyl-estra-1,3,5(10)-trien-17α-ol in 27 ml Ethanol wurden nacheinander 2.13 g Kaliumfluorid und 0.55 ml Wasser gegeben und 18 Stunden bei Raumtemperatur gerührt. Nach dem Einengen des Lösungsmittels wurde der Rückstand in Essigester aufgenommen und 2x mit Wasser und Kochsalzlösung gewaschen. Nach der Filtration der organischen Phase und Entfernen des Lösungsmittels am Rotationsverdampfer lieferte die anschließende Flash-Chromatographie an Kieselgel (Hexan / Essigester) 7α-(5-Chlorpentyl)-11β-fluor-17β-methyl-estra-1,3,5(10)-trien-3,17α-diol (2,74 g).
H-NMR: 400 MHz, CDCl₃, δ = 7.20 (d, 1H), 6.67 (m, 1H), 6.57 (s (br.), 1H), 5.60 (d, 1H), 4.71 (s, 1H), 3.51 (t, 2H), 2.92 (dd, 1H), 2.71 (d, 1H), 2.54 (dd, 1H), 0.88 (d, 3H).

### 5.1.2 4-Brom-7α-(5-chlorpentyl)-11β-fluor-17β-methyl-estra-1,3,5(10)-trien-3,17α-diol

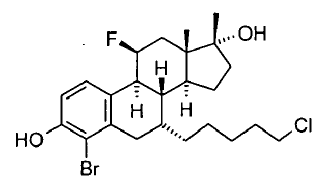

Bei 0°C wurde zu einer Lösung von 2.5 g 7α-(5-Chlorpentyl)-11β-fluor-17β-methyl-estra-1,3,5(10)-trien-3,17α-diol in 278 ml Chloroform die Lösung von 1.17 g N-Brom-Succinimid in 167 ml Chloroforn gegeben, anschließend für 30 Minuten bei dieser Temperatur gerührt. Anschließend ließ man langsam auf Raumtemperatur erwärmen und rührte noch weitere 30 Minuten nach. Nach dem Einengen des Reaktionsgemisches wurde der Rückstand in Diethylether aufgenommen, 2x mit Wasser gewaschen, über Magnesiumsulfat getrocknet, abfiltriert und erneut zur Trockne eingeengt. Flash-Chromatographie an Kieselgel (Hexan / Essigester) lieferte 4-Brom-7α-(5-chlorpentyl)-11β-fluor-17β-methyl-estra-1,3,5(10)-trien-3,17α-diol (1.86 g).
H-NMR: 400 MHz, CDCl₃, δ = 7.25 (d, 1H), 6.91 (m, 1H), 5.60 (d, 1H), 5.56 (s, 1H), 3.52 (t, 2H), 2.95 (d, 1H), 1.26 (s, 3H), 0.87 (d, 3H).

### 5.2 Herstellung des Endproduktes

Bei Raumtemperatur wurde zu einer Lösung von 355 mg 4-Brom-7α-(5-chlorpentyl)-11β-fluor-17β-methyl-estra-1,3,5(10)-trien-3,17α-diol in 2.18 ml Dimethylformamid 77.1 mg Natriumcarbonat, 218.1 mg Natriumiodid und 270 mg (8,8,9,9,9-Pentafluornonyl)-methylamin gegeben. Anschließend ließ man für 5 Stunden bei 80°C rühren. Nach dem Entfernen des Lösungsmittels wurde der Rückstand in Essigester aufgenommen, mit Wasser und halbgesättigter Natriumhydrogencarbonat-Lösung gewaschen, mit Magnesiumsulfat getrocknet, abfiltrierte und das Lösungsmittel am Rotationsverdampfer entfernt. Flash-Chromatographie an Kieselgel (Aminphase; Hexan / Essigester) lieferte 4-Brom-11β-fluor-17β-methyl-7α-[5-[methyl(8,8,9,9,9-pentafluornonyl)amino]pentyl]-estra-1,3,5(10)-trien-3,17α-diol, das aus Diethylether umkristallisiert wurde (228 mg).
H-NMR: 400 MHz, CDCl₃, δ = 7.24 (d, 1H), 6.90 (d, 1H), 5.59 (d, 1H), 2.96 (d, 1H), 2.18 (s, 3H), 1.26 (s, 3H), 0.87 (d, 3H).

### Beispiel 6

### 4-Brom-11β-fluor-17β-methyl-7α-[5-[(2R)-2-(7,7,8,8,8-pentafluoroctyl)-1-pyrrolidinyl]pentyl]-estra-1,3,5(10)-trien-3,17α-diol

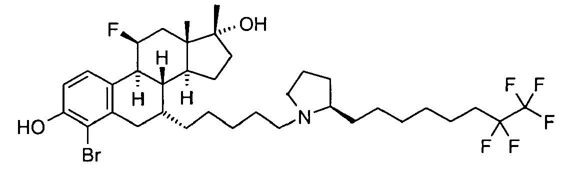

In Analogie zu Schritt 5.2 des Beispiels 5 lieferten 223 mg 4-Brom-7α-(5-chlorpentyl)-11β-fluor-17β-methyl-estra-1,3,5(10)-trien-3,17α-diol mit 187 mg (*R*)-2-(7,7,8,8,8-Pentafluor-octyl)-pyrrolidin als Amin (Verbindung 2.1.1) nach der Flash-Chromatographie (Aminphase; Hexan / Essigester) 4-Brom-11β-fluor-17β-methyl-7α-[5-[(2R)-2-(7,7,8,8,8-pentafluoroctyl)-1-pyrrolidinyl]pentyl]-estra-1,3,5(10)-trien-3,17α-diol (250 mg).
H-NMR: 400 MHz, CDCl₃, δ = 7.24 (d, 1H), 6.90 (d, 1H), 5.60 (d, 1H), 3.13 (m, 1H), 2.96 (d, 1H), 1.26 (s, 3H), 0.86 (d, 3H).

### Beispiel 7

### 4-Chlor-11β-fluor-17β-methyl-7α-[5-[(2R)-2-(7,7,8,8,8-pentafluoroctyl)-1-pyrrolidinyl]pentyl]-estra-1,3,5(10)-trien-3,17α-diol

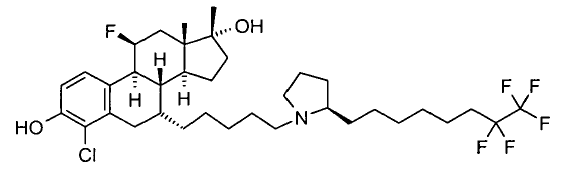

### 7.1 Herstellung der Intermediate

### 7.1.1 4-Chlor-7α-(5-chlorpentyl)-11β-fluor-17β-methyl-estra-1,3,5(10)-trien-3,17α-diol

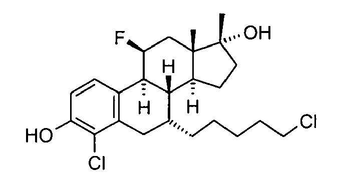

Bei Raumtemperatur wurde zu einer Lösung von 262 mg 3-(*tert*-Butyldimethylsilyloxy)-4-chlor-7α-(5-chlorpentyl)-11β-fluor-17β-methyl-estra-1,3,5(10)-trien-17α-ol in 8 ml Ethanol nacheinander 164 mg Kaliumfluorid und 42 µl Wasser gegeben und 24 Stunden bei Raumtemperatur gerührt. Nach dem Einengen des Lösungsmittels wurde der Rückstand in Essigester aufgenommen und mit Wasser gewaschen. Nach der Filtration der organischen Phase und Entfernen des Lösungsmittels am Rotationsverdampfer lieferte die anschließende Flash-Chromatographie an Kieselgel (Hexan / Essigester) 4-Chlor-7α-(5-chlorpentyl)-11β-fluor-17β-methyl-estra-1,3,5(10)-trien-3,17α-diol (94 mg).
H-NMR: 400 MHz, CDCl₃, δ = 7.21 (d, 1H), 6.90 (d, 1H), 5.60 (d, 1H), 5.55 (s (br.), 1H), 3.52 (t, 2H), 2.98 (d, 1H), 1.26 (s, 3H), 0.87 (d, 3H).

### 7.2 Herstellung des Endproduktes

In Analogie zu Schritt 5.2 des Beispiels 5 lieferten 206 mg 4-Chlor-7α-(5-chlorpentyl)-11β-fluor-17β-methyl-estra-1,3,5(10)-trien-3,17α-diol mit 209 mg (R)-2-(7,7,8,8,8-Pentafluor-octyl)-pyrrolidin als Amin (Verbindung 2.1.1) nach der wiederholter Flash-Chromatographie (Kieselgel gefolgt von Aminphase; Hexan / Essigester) 4-Chlor-11β-fluor-17β-methyl-7α-[5-[(2R)-2-(7,7,8,8,8-pentafluoroctyl)-1-pyrrolidinyl]pentyl]-estra-1,3,5(10)-trien-3,17α-diol (165 mg).
H-NMR: 400 MHz, CDCl₃, δ = 7.20 (d, 1H), 6.90 (d, 1H), 5.60 (d, 1H), 3.12 (m, 1H), 2.99 (d, 1H), 1.26 (s, 3H), 0.876 (s (br.), 3H).

### Beispiel 8

### 4-Brom-11β-fluor-17β-methyl-7α-[5-[(2R)-2-(6,6,7,7,7-pentafluorheptyl)-1-pyrrolidinyl]pentyl]-estra-1,3,5(10)-trien-3,17α-diol

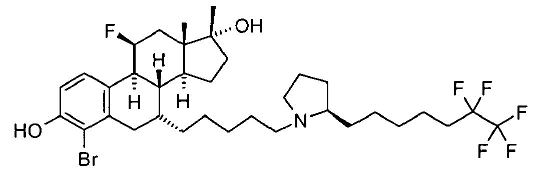

In Analogie zu Schritt 5.2 des Beispiels 5 lieferten 255 mg 4-Brom-7α-(5-chlorpentyl)-11β-fluor-17β-methyl-estra-1,3,5(10)-trien-3,17α-diol mit 178 mg (*R*)-2-(7,7,8,8,8-Pentafluor-heptyl)-pyrrolidin als Amin nach der Flash-Chromatographie (Kieselgel; Hexan / Essigester) 4-Brom-11β-fluor-17β-methyl-7α-[5-[(2R)-2-(6,6,7,7,7-pentafluorheptyl)-1-pyrrolidinyl]pentyl]-estra-1,3,5(10)-trien-3,17α-diol (287 mg).
H-NMR: 400 MHz, CDCl₃, δ = 7.24 (d, 1H), 6.90 (d, 1H), 5.60 (d, 1H), 3.12 (m, 1H), 2.96 (d, 1H), 1.26 (s, 3H), 0.86 (d, 3H).

### Beispiel 9

### 4-Chlor-11β-fluoro-17β-methyl-7α-[5-[(2R)-2-(6,6,7,7,7-pentafluorheptyl)-1-pyrrolidinyl]pentyl]-estra-1,3,5(10)-trien-3,17α-diol

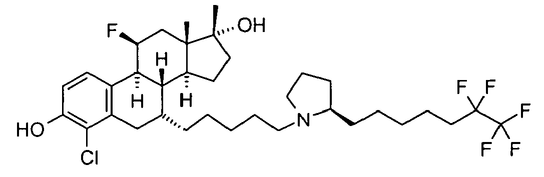

### 9.1 Herstellung der Intermediate

### 9.1.1 4-Chlor-7α-(5-chlorpentyl)-11β-fluor-17β-methyl-estra-1,3,5(10)-trien-3,17α-diol

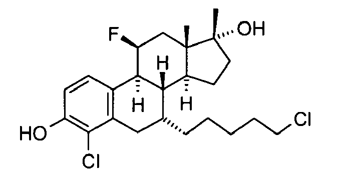

Alternativ zur Vorschrift gemäß Schritt 7.1.1 des Beispiels 7 ließ sich 4-Chlor-7α-(5-chlorpentyl)-11β-fluor-17β-methyl-estra-1,3,5(10)-trien-3,17α-diol herstellen, in dem zu einer Lösung von 325 mg -(5-Chlorpentyl)-11β-fluor-17β-methyl-estra-1,3,5(10)-trien-3,17α-diol in 6 ml Dimethylformamid unter Stickstoffatmosphäre 239 mg 2,3,4,5,6,6-Hexachlor-2,4-cyclohexadien-1-on gegeben wurde und 24 Stunden bei Raumtemperatur gerührt wurde. Danach erfolgte eine weitere Zugabe von 12 mg 2,3,4,5,6,6-Hexachlor-2,4-cyclohexadien-1-on. Nachdem weitere 56 Stunden gerührt wurde, wurde das Reaktionsgemisch auf Wasser gegeben und mit Essigester extrahiert. Nach dem Trocknen und Einengen der organischen Phase lieferte die anschließende Flash-Chromatographie an Kieselgel (Hexan / Essigester) 4-Chlor-7α-(5-chlorpentyl)-11β-fluor-17β-methyl-estra-1,3,5(10)-trien-3,17α-diol (240 mg).
H-NMR: 400 MHz, CDCl₃, wie unter 7.1.1 angegeben.

### 9.2 Herstellung des Endproduktes

In Analogie zu Schritt 5.2 des Beispiels 5 lieferten 240 mg 4-Chlor-7α-(5-chlorpentyl)-11β-fluor-17β-methyl-estra-1,3,5(10)-trien-3,17α-diol mit 238 mg (R)-2-(7,7,8,8,8-Pentafluor-heptyl)-pyrrolidin als Amin nach wiederholter Flash-Chromatographie (Kieselgel gefolgt von Aminphase; Hexan / Essigester) 4-Chlor-11β-fluoro-17β-methyl-7α-[5-[(2R)-2-(6,6,7,7,7-pentafluorheptyl)-1-pyrrolidinyl]pentyl]-estra-1,3,5(10)-trien-3,17α-diol (205 mg).
H-NMR: 400 MHz, CDCl₃, δ = 7.20 (d, 1H), 6.89 (d, 1H), 5.60 (d, 1H), 3.14 (m, 1H), 2.99 (d, 1H), 1.26 (s, 3H), 0.87 (d, 3H).

### Beispiel 10

### 2,4-Dichlor-11β-fluor-17β-methyl-7α-[5-[(2R)-2-(7,7,8,8,8-pentafluoroctyl)-1-pyrrolidinyl]pentyl]-estra-1,3,5(10)-trien-3,17α-diol

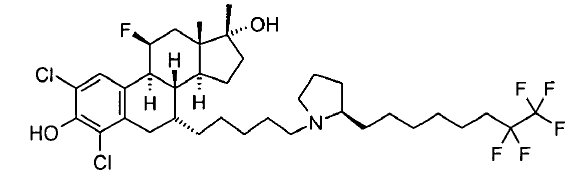

### 10.1 Herstellung der Intermediate

### 10.1.1 7α-(5-Chlorpentyl)-2,4-dichlor-11β-fluor-17β-methyl-estra-1,3,5(10)-trien-3,17α-diol

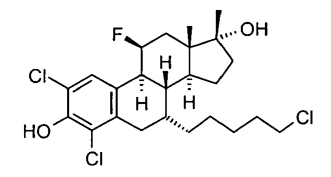

Die in Schritt 4.4.1 des Beispiels 4 beschriebene Chlorierung lieferte neben der gewünschten reinen Zielstruktur nach Flash-Chromatographie auch geringe Mengen der Mischung aus 3-(*tert*-Butyldimethylsilyloxy)-4-chlor-7α-(5-chlorpentyl)-11β-fluor-17β-methyl-estra-1,3,5(10)-trien-17α-ol und 3-(*tert*-Butyldimethylsilyloxy)-7α-(5-chlorpentyl)-2,4-dichlor-11 β-fluor-17β-methyl-estra-1,3,5(10)-trien-17α-ol, das gemäß der im Beispiel 7.1.1 beschriebenen Methode de-silyliert wurde. Flash-Chromatographie an Kieselgel (Hexan / Essigester) lieferte 7α-(5-Chlorpentyl)-2,4-dichlor-11β-fluor-17β-methyl-estra-1,3,5(10)-trien-3,17α-diol (76 mg).
H-NMR: 400 MHz, CDCl₃, δ = 7.28 (s, 1H), 5,82 (s (br.) 1H), 5.53 (d, 1H), 3.51 (t, 2H), 2.97 (d, 1H), 1.26 (s, 3H), 0.86 (s, 3H).

### 10.2 Herstellung des Endproduktes

In Analogie zu Schritt 5.2 des Beispiels 5 lieferten 76 mg 7α-(5-Chlorpentyl)-2,4-dichlor-11β-fluor-17β-methyl-estra-1,3,5(10)-trien-3,17α-diol mit 65 mg (R)-2-(7,7,8,8,8-Pentafluor-octyl)-pyrrolidin als Amin (Verbindung 2.1.1) nach Flash-Chromatographie (Kieselgel; Hexan / Essigester) gefolgt von präp. HPLC und Entfernung von Ameisensäurespuren mittels Natriumhydrogencarbonatlösung 2,4-Dichlor-11β-fluor-17β-methyl-7α-[5-[(2R)-2-(7,7,8,8,8-pentafluoroctyl)-1-pyrrolidinyl]pentyl]-estra-1,3,5(10)-trien-3,17α-diol (16 mg).
H-NMR: 400 MHz, CDCl₃, δ = 7.27 (s, 1H), 5.54 (d, 1H), 3.13 (m, 1H), 2.98 (d, 1H), 1.26 (s, 3H), 0.86 (d, 3H).

### B. Herstellung der 17α-Alkyl-Vergleichsverbindungen

### V1:

### 11β-Fluor-17α-methyl-7α-[5-[methyl(8,8,9,9,9-pentafluornonyl)amino]pentyl]-estra-1,3,5(10)-trien-3,17β-diol CAS [536975-64-1]

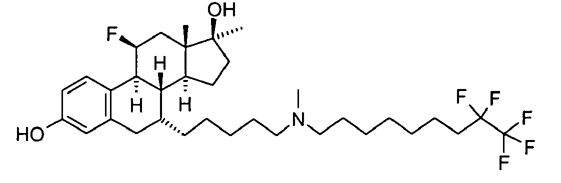

Eine Lösung von 19.5 g 7α-(5-Brompentyl)-11β-fluor-17α-methyl-estra-1,3,5(10)-trien-3,17β-diol (WO 2003/045972, Seite 27, 2.2a) in 200 ml absolutem Dimethylformamid wurde mit 12.75 g Methyl-(8,8,9,9,9-pentafluornonyl)-amin und 5.47 g Natriumcarbonat, versetzt. Anschließend ließ man für 5.5 Stunden bei 80°C rühren. Nach dem Entfernen des Lösungsmittels wurde der Rückstand in Essigester aufgenommen, mit Wasser und halbgesättigter Natriumhydrogencarbonat-Lösung gewaschen, mit Magnesiumsulfat getrocknet, abfiltrierte und das Lösungsmittel am Rotationsverdampfer entfernt. Flash-Chromatographie an Kieselgel (Aminphase; Hexan / Essigester) lieferte 11β-Fluor-17α-methyl-7α-[5-[methyl(8,8,9,9,9-pentafluornonyl)amino]pentyl]-estra-1,3,5(10)-trien-3,17β-diol, das aus Diethylether umkristallisiert wurde (14.5 g);
H-NMR: 300 MHz, CDCl₃, δ = 7.16 (d, 1H), 6.64 (d, 1H), 6.54 (d, 1H), 5.56 (d, 1H), 2.90 (d, 1H), 2.69 (d, 1H), 2.40 - 2.34 (m, 4H), 2.25 (s, 3H), 1.06 (d, 3H).

### V2:

### 11β-Fluor-17α-methyl-7α-[5-[(2R)-2-(7,7,8,8,8-pentafluoroctyl)-1-pyrrolidinyl]pentyl]-estra-1,3,5(10)-trien-3,17β-diol

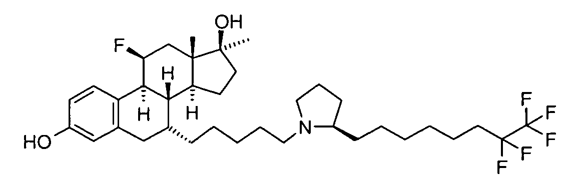

Eine Lösung von 3.45 g 7α-(5-Chlorpentyl)-11β-fluor-17α-methyl-estra-1,3,5(10)-trien-3,17β-diol in 45 ml absolutem Dimethylformamid wurde mit 3.0 g (2R)2-(7,7,8,8,8-Pentafluoroctyl)-pyrrolidine, 2.01 g Natriumcarbonat und 2.53 g Natriumiodid versetzt. Anschließend ließ man für 5 Stunden bei 100°C rühren. Nach dem Entfernen des Lösungsmittels wurde der Rückstand in Essigester aufgenommen, mit Wasser und halbgesättigter Natriumhydrogencarbonat-Lösung gewaschen, mit Natriumsulfat getrocknet, abfiltrierte und das Lösungsmittel am Rotationsverdampfer entfernt. Flash-Chromatographie an Kieselgel (Aminphase; Hexan / Essigester) lieferte 11β-Fluor-17α-methyl-7α-[5-[methyl(8,8,9,9,9-pentafluornonyl)amino]pentyl]-estra-1,3,5(10)-trien-3,17β-diol, das aus Diethylether umkristallisiert wurde (4.5 g).
H-NMR: 600 MHz, CDCl₃, δ = 7.17 (d, 1H), 6.66 (d, 1H), 6.56 (d, 1H), 5.56 (d, 1H), 2.89 (d, 1H), 2.71 (d, 1H), 1.06 (d, 3H).

### V3:

### 11β-Fluor-17α-methyl-7α-[5-[(2R)-2-(6,6,7,7,7-pentafluorheptyl)-1-pyrrolidinyl]pentyl]-estra-1,3,5(10)-trien-3,17β-diol

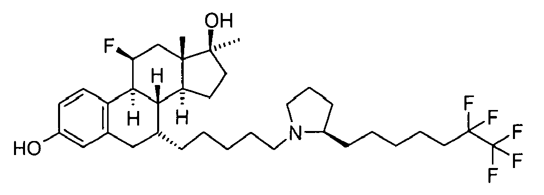

Eine Lösung von 10 g 7α-(5-Chlorpentyl)-11β-fluor-17α-methyl-estra-1,3,5(10)-trien-3,17β-diol in 50 ml absolutem Dimethylformamid wurde mit 11.83 g (2R)2-(6,6,7,7,7-Pentafluorheptyl)-pyrrolidine, 2.6 g Natriumcarbonat und 7.33 g Natriumiodid versetzt. Anschließend ließ man für 18 Stunden bei 80°C rühren. Nach dem Entfernen des Lösungsmittels wurde der Rückstand in Essigester aufgenommen, mit Wasser und halbgesättigter Natriumhydrogencarbonat-Lösung gewaschen, mit Natriumsulfat getrocknet, abfiltrierte und das Lösungsmittel am Rotationsverdampfer entfernt. Flash-Chromatographie an Kieselgel (Aminphase; Hexan / Essigester) lieferte 11β-Fluor-17α-methyl-7α-[5-[methyl(7,7,8,8,8-pentafluornonyl)amino]pentyl]-estra-1,3,5(10)-trien-3,17β-diol, das aus Diethylether umkristallisiert wurde (9.84 g).
H-NMR: 600 MHz, CDCl₃, δ = 7.15 (d, 1H), 6.61 (d, 1H), 6.51 (d, 1H), 5.56 (d, 1H), 2.88 (d, 1H), 2.72 (d, 1H), 1.05 (d, 3H).

### C. Charakterisierung

### C.1 Beschreibungen der charakterisierenden Versuche

### C.1.1 Metabolische Stabilität:

Die metabolische Stabilität von Prüfsubstanzen wurden durch Inkubationen in einer Suspension mit humanen Lebermikrosomen, welche auf einen Proteingehalt von 0.5 mg/mL eingestellt waren, bei einer Konzentrationen von 0.3µM bestimmt. Das Inkubationsvolumen lag bei 3.03 ml, wobei 2.4ml einer Mikrosomensuspension in Phosphatpuffer bei pH 7.4 (Natrium - Phosphatpuffer 100 mM (NaH₂PO₄x H₂O + Na₂HPO₄x 2H₂O) vorgelegt wurden, welche durch Zugabe von 0.6 ml eines Cofaktormixes (bestehend aus 1.2 mg NADP, 3 IU Glucose-6-Phosphat Dehydrogenase, 14.6 mg Glucose-6-Phosphat und 4.9 mg MgCl₂ in Phosphatpuffer, pH 7.4) aktiviert wurde. Durch Zugabe von 30µL einer Prüfsubstanzstammlösung wurde der Assay gestartet, wobei die Stammlösung so zusammengesetzt war, dass die Lösungsmittelkonzentrationen während der Inkubation für DMSO <0.2% und für Methanol < 1% lag. Die Inkubationen wurden bei einer Inkubationstemperatur von 37°C über einen Zeitraum von 60 Minuten durchgeführt, wobei die Mikrosomen durch kontinuierliches Rühren (Tec Control Shaker RS 485 bei 300 UpM) in einer homogenen Suspension gehalten wurden. Zu 6 verschiedenen Zeitpunkten (2, 8, 16, 30, 45 & 60 min) wurden Aliquots von jeweils 250 µl gezogen und sofort in dasselbe Volumen eiskaltem Methanol gegeben und abgedeckt. Die Proben wurden über Nacht bei - 20°C ausgefroren und danach für 15 min bei 3000 UpM zentrifugiert bevor 100 µl des klaren Überstandes zur Konzentrationsbestimmung abgenommen wurde. Die Analytik erfolgte durch ein Agilent 1200 HPLC-System mit angeschlossener LCMS/MS Detektion.

Aus den abnehmenden Konzentrationen über die Zeit wurde die Halbwertszeit der Prüfsubstanz in dem oben beschriebenen mikrosomalen Inkubationsgemisch bestimmt, aus dem wiederum die "intrinsische Clearance", die maximale Geschwindigkeit der Lebermikrosomen, die Prüfsubstanz metabolisch zu eliminieren errechnet wurde. Aus dieser "intrinsischen Clearance" wiederum lässt sich unter Hinzunahme verschiedener physiologischer Kenngrößen eine maximale metabolische Clearance im Menschen abschätzen, wobei durch das oben genannte Prinzip ausschließlich Phase I metabolische Reaktionen abgebildet werden können (typischer Weise: oxidoreduktive Reactions durch Zytochrom P450 Enzyme oder der Flavinmonooxigenase, sowie hydrolytische Reaktionen von Esterasen und Amidasen). Physiologische Kenngrößen sind: humaner Leberblutfluss: 1.3 I/kg/h ; spezifisches Lebergewicht (pro kg Körpergewicht): 21g/kg; mikrosomaler Proteingehalt: 40mg/g Leber. Weiterhin wurde unter den Annahmen, dass (i) die Absorption der Prüfsubstanz im Menschen 100% beträgt, und (ii) die abgeschätzte metabolische Clearance sich als first pass berechnen lässt, eine maximale orale Bioverfügbarkeit (Fmax) abgschätzt.
Formeln & Kurzerklärungen: CL intrinsic-apparent [in ml/(min*mg protein)]: spiegelt die Eliminationskonstante kel (in min-1) der Prüfsubstanz in dem Inkubationsansatz wieder, geteilt durch den mikrosomalen Proteingehalt (40 mg/ml). CL intrinsic [in l(h*kg)]: maximale Fähigkeit der Leber (Microsomen) die Prüfsubstanz metabolisch zu eliminieren, wenn der Leberblutfluss nicht der limitierende Faktor ist (kel*Lebergewicht) / Anteil der Leber (Mikrosomaler Gehalt) in der Inkubation. CL blood-well stirred [in l/(h*kg)]: abgeschätzte Blutclearance (via Phase 1 Metabolismus): (QH*CL intrinsic) / (QH+CL intrinsic). Fmax [in %]: maximale Bioverfügbarkeit nach oraler Applikation derPrüfsubstanz: (1-CL blood-well stirred/QH)*100.

### C.1.2 Bioverfügbarkeit

Die Bestimmung der intragastralen Bioverfügbarkeit von Prüfsubstanzen wurde in weiblichen wachen Hunden mit einem Körpergewicht von minimal 5kg bis maximal 12kg durchgeführt. Hierzu wurden die Prüfsubstanzen sowohl für die intravenösen 15min-Infusion als auch für die intragastrale Applikation in gelöster Form appliziert wobei verträgliche Lösungsvermittler wie PEG400 und/oder Ethanol in verträglicher Menge verwendet wurden.

### Intravenöse Applikation:

Die Prüfsubstanzen wurden bei einer geringen Dosis von 0.2 - 1 mg/kg über einen Zeitraum von 15 min infundiert. Dabei wurden zu den Zeitpunkten 5min, 10min, 15min (bzw. kurz vor Infusionsende), 20min, 30min, 45min, 1 h, 2h, 4h, 6h, 8h, 24h (bezogen auf Infusionsbeginn) ca. 1 - 1.5ml Blutproben entnommen. Die Blutproben wurden ohne Schütteln in Lithium-Heparinröhrchen (Monovetten® von Sarstedt) aufbewahrt und für 15 min bei 3000 Upm zentrifugiert. Ein Aliquot von 100µL wurde dem Überstand (Plasmawasser) entnommen und durch Zugabe von 400 µl kaltem ACN gefällt. Die gefällten Proben wurden über Nacht bei - 20°C ausgefroren, danach wiederum für 15min bei 3000UpM zentrifugiert bevor 150µL des klaren Überstandes zur Konzentrationsbestimmung abgenommen wurde. Die Analytik erfolgte durch ein Agilent 1200 HPLC-System mit angeschlossener LCMS/MS Detektion.Berechnung der PK Parameter (via PK Berechnungssoftware, z.B. WinNonLin^{®}): CLplasma: Gesamtplasmaclearance der Prüfsubstanz (in l/kg/h); CLblood:
Gesamtblutclearance der Prüfsubstanz (in l/kg/h), wobei (CLblood = CLplasma*Cp/Cb); Vss: Apparentes Verteilungsvolumen beim steady state (in l/kg); T1/2: Halbwertszeit innerhalb eines spezifizierten Intervalls (hier: terminale T1/2, in h); AUCnorm: Fläche unter dem Plasmakonzentrationszeitprofil vom Zeitpunkt Null bis zur Unendlichkeit extrapoliert geteilt durch die Dosis (in kg*l/h); AUC(0-tn)norm: Integrierte Fläche unter dem Plasmakonzentrationszeitprofil vom Zeitpunkt Null bis zum letzten Zeitpunkt, zu dem eine Plasmakonzentration messbar war, geteilt durch die Dosis (in kg*l/h); Cmax: Maximale Konzentration der Prüfsubstanz im Plasma (in µg/l); Cmax,norm: Maximale Konzentration der Prüfsubstanz im Plasma geteilt durch die Dosis (in kg/l); Cb/Cp: Verhältnis der Blut zu Plasma Konzentrationsverteilung.

### Intragastrale Applikation:

Die Prüfsubstanzen wurden bei einer geringen Dosis von 1 -2 mg/kg über eine Schlauchsonden intragastral als Bolus an nüchterne weibliche Hunde appliziert. Zu den Zeitpunkten 15min, 30min, 45min, 1 h, 2h, 4h, 6h, 8h, 24h (bezogen auf Infusionsbeginn) wurden ca. 1-1.5mL Blut entnommen. Die Blutproben wurden ohne Schütteln in Lithium-Heparinröhrchen (Monovetten® von Sarstedt) aufbewahrt und für 15min bei 3000Upm zentrifugiert. Ein Aliquot von 100µl wurde dem Überstand (Plasmawasser) entnommen und durch Zugabe von 400µL kaltem ACN gefällt. Die gefällten Proben wurden über Nacht bei - 20°C ausgefroren, danach für 15min bei 3000UpM zentrifugiert bevor 150µl des klaren Überstandes zur Konzentrationsbestimmung abgenommen wurde. Die Analytik erfolgte durch ein Agilent 1200 HPLC-System mit angeschlossener LCMS/MS Detektion

Berechnung der PK Parameter (via PK Berechnungssoftware, z.B. WinNonLin^{®}): AUCnorm: Fläche unter dem Plasmakonzentrationszeitprofil vom Zeitpunkt Null bis zur Unendlichkeit extrapoliert geteilt durch die Dosis (in kg*L/h); AUC(0-tn)norm: Integrierte Fläche unter dem Plasmakonzentrationszeitprofil vom Zeitpunkt Null bis zum letzten Zeitpunkt, zu dem eine Plasmakonzentration messbar war, geteilt durch die Dosis (in kg*l/h); Cmax: Maximale Konzentration der Prüfsubstanz im Plasma (in µg/L); Cmax,norm: Maximale Konzentration der Prüfsubstanz im Plasma geteilt durch die Dosis (in kg/l); T1/2: Halbwertszeit innerhalb eines spezifizierten Intervalls (hier: terminale T1/2, in h); Fobs%: beobachtete orale Bioverfügbarkeit, AUC(0-tn)norm nach i.g. Gabe geteilt durch AUC(0-tn)norm nach i.v. Gabe. tmax: Zeitpunkt, zudem die maximale Konzentration der Prüfsubstanz im Plasma gemessen wird.

### C.2 Ergebnisse

Tabelle 2 zeigt die Ergbnisse der PK-Charakterisierung
(V1-3: direkte Analoga d.h. Unterschied nur in Stereoisomerie in Position 17).

**Tab. 2**

| Verbindung [Bsp.] | Fₘₐₓ (Mikros.) [%] | F_{obs} (Hund) [%] |
|---|---|---|
| 1 | 39 | 16 |
| V1 | 9 | 2.8 |
| 2 | 36 | 13 |
| V2 | 9 | 2 |
| 3 | 40 | 13 |
| V3 | 8 | 3.1 |
| 4 | 39 | |
| 5 | 55 | |
| 6 | 34 | |
| 7 | 47 | 14 |
| 8 | 48 | |
| 9 | 42 | |
| 10 | 34 | |

Die erfindungsgemäßen Verbindungen mit einer 17β-Gruppe sind den direkten Analoga überlegen, weil sie eine erhöhte metabolische Stabilität in humanen Mikrosomen und eine erhöhte Bioverfügbarkeit im Hund nach intragastraler gabe aufweisen.

## Patentansprüche

1. Verbindungen der allgemeinen Formel (I) 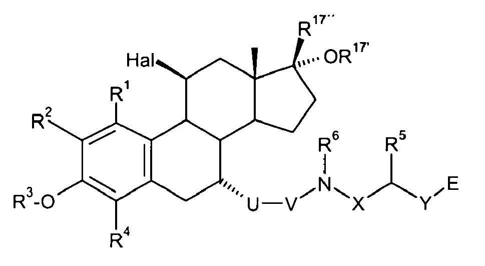 in der
**Hal** für Fluor oder Chlor steht, das in 11 β-Position an das Estratriengrundgerüst gebunden ist; und
**R¹, R² und R⁴** unabhängig voneinander für Wasserstoff, Fluor, Chlor oder Brom stehen, und
**R³** für Wasserstoff oder einen C₁-C₄-Alkyl- oder C₁-C₄-Alkanoylrest steht, und
**R^{17'}** für Wasserstoff oder einen C₁-C₄-Alkyl- oder C₁-C₄-Alkanoylrest steht,
**R^{17"}** für einen gegebenenfalls ein- oder mehrfach fluorierten C₁-C₄-Alkyl-, C₂-C₄- Alkenyl- oder C₂ - C₄-Alkinylrest steht, wobei **R^{17"}**-O in 17α-Position und **R^{17"}** in 17β-Position an das Estratriengrundgerüst gebunden sind; und
**U** für einen gerad- oder verzweigtkettigen C₁-C₁₃-Alkylen-, C₂-C₁₃-Alkenylen- oder C₂-C₁₃-Alkinylenrest steht, oder für die Gruppe **A-B** wobei **A** an das Estratriengrundgerüst gebunden ist und einen über -CH₂- an das Estratriengrundgerüst gebundenen Benzylidenrest, einen Phenylenrest oder einen über die Alkylgruppe an das Estratriengrundgerüst gebundenen C₁-C₃- Alkylen-Phenylenrest darstellt und **B** für einen gerad- oder verzweigtkettigen C₁-C₁₃-Alkylen-, C₂-C₁₃-Alkenylen- oder C₂-C₁₃-Alkinylenrest steht und wobei **A** und **B** auch über ein O-Atom miteinander verbunden sein können,
**V** für eine Methylen- oder eine -C(O)-Gruppe steht und
**X** für eine Bindung oder eine C₁-C₃-Alkylengruppe steht und
**R⁵** für Wasserstoff oder einen C₁-C₄-Alkyl-, C₂-C₄-Alkenyl- oder C₂-C₄-Alkinylrest steht
**R⁶** für Wasserstoff oder die Gruppe -CH₂-**R⁷** oder C(O)-**R⁷** steht, worin **R⁷** steht für Wasserstoff oder einen gerad- oder verzweigtkettigen, nichtfluorierten oder
**R⁵ und R⁶** zumindest teilweise fluorierten C₁-C₆-Alkyl-, C₂-C₆-Alkenyl- oder C₂-C₆- Alkinylrest, der einfach oder mehrfach mit Hydroxy substituiert sei kann, oder zusammen mit X und dem Stickstoffatom der Seitenkette einen 4 bis 6 gliedrigen Heterocyclylring bilden, der zusätzlich zum Stickstoffatom der Seitenkette ein weiteres Heteroatom aufweisen kann und/oder eine Carbonylgruppe enthalten kann,
**Y** für eine C₅-C₈-Alkylengruppe steht,
**E** für einen C₁-C₄-Perfluoroalkylrest oder für einen Phenylrest steht, der ein- bis fünffach substituiert ist mit Halogen oder -CF₃,
sowie deren Enantiomere und Diastereomere, deren Salze, Solvate und Salze der Solvate.

2. Verbindungen gemäß Anspruch 1, in der
**Hal** für Fluor steht, und
**R¹, R² und R⁴** unabhängig voneinanderfür Wasserstoff, Chlor oder Brom stehen, und
**R³** für Wasserstoff, einen Methyl- oder Acetylrest steht, und
**R⁵** für Wasserstoff oder einen C₁-C₃-Alkylrest steht, und
**R⁶** für Wasserstoff oder -CH₂-**R⁷** steht, worin **R⁷** für Wasserstoff oder einen Methyl- oder Ethylrest steht, oder
**R⁵ und R⁶** zusammen mit dem Stickstoffatom der Seitenkette einen 5-gliedrigen Heterocyclylring bilden, der zusätzlich zum Stickstoffatom der Seitenkette ein weiteres Heteroatom aufweisen kann und/oder eine Carbonylgruppe enthalten kann, und
**R^{17'}** für Wasserstoff, einen Methylrest oder Acetylrest steht, und
**R^{17"}** für einen Methyl-, Ethinyl- oder Trifluormethylrest steht, und
**U** für einen Butylen-, Pentylen-, Hexylen- oder Heptylenrest steht, und
**V** für eine Methylengruppe steht, und
**X** für eine Bindung oder eine Methylengruppe steht, und
**Y** für eine Bindung oder eine C₅-C₇-Alkylengruppe steht, und
**E** für -C₂F₅, -C₃F₇, -C₄F₉ oder für einen Trifluormethylphenylrest steht,
sowie deren Enantiomere und Diastereomere, deren Salze, Solvate und Salze der Solvate.

3. Verbindungen gemäß Anspruch 1 oder 2, in der
**R¹** für Wasserstoff steht, und
**R²** für Wasserstoff oder Chlor steht, und
**R⁴** für Wasserstoff, Chlor oder Brom steht,
sowie deren Enantiomere und Diastereomere, deren Salze, Solvate und Salze der Solvate.

4. Verbindungen gemäß einem der Ansprüche 1 bis 3, in der
**R³** für Wasserstoff steht,
sowie deren Enantiomere und Diastereomere, deren Salze, Solvate und Salze der Solvate.

5. Verbindungen gemäß einem der Ansprüche 1 bis 4, in der
**Hal** für Fluor steht,
sowie deren Enantiomere und Diastereomere, deren Salze, Solvate und Salze der Solvate.

6. Verbindungen gemäß einem der Ansprüche 1 bis 5, in der
**R⁵** für Wasserstoff und **R⁶** für eine Methylgruppe steht oder
R⁵ und R⁶ zusammen mit dem Stickstoffatom der Seitenkette einen Pyrrolidinring bilden,
sowie deren Enantiomere und Diastereomere, deren Salze, Solvate und Salze der Solvate.

7. Verbindungen gemäß einem der Ansprüche 1 bis 6, in der
**R^{17'}** für Wasserstoff steht und **R^{17"}** für einen Methylrest steht,
sowie deren Enantiomere und Diastereomere, deren Salze, Solvate und Salze der Solvate.

8. Verbindungen gemäß einem der Ansprüche 1 bis 7, in der
**U** für einen n-Butylenrest steht,
sowie deren Enantiomere und Diastereomere, deren Salze, Solvate und Salze der Solvate.

9. Verbindungen gemäß einem der Ansprüche 1 bis 8, in der
V für eine Methylengruppe steht,
sowie deren Enantiomere und Diastereomere, deren Salze, Solvate und Salze der Solvate.

10. Verbindungen gemäß einem der Ansprüche 1 bis 9, in der
**X** für eine Bindung steht,
sowie deren Enantiomere und Diastereomere, deren Salze, Solvate und Salze der Solvate.

11. Verbindungen gemäß einem der Ansprüche 1 bis 10, in der
**Y** für eine n-Pentylen- oder n-Hexylengruppe steht,
sowie deren Enantiomere und Diastereomere, deren Salze, Solvate und Salze der Solvate.

12. Verbindungen gemäß einem der Ansprüche 1 bis 11, in der
**E** für -C₂F₅ steht,
sowie deren Enantiomere und Diastereomere, deren Salze, Solvate und Salze der Solvate.

13. Verbindungen gemäß Formel (I) nach Anspruch 1, in der
**R¹** für Wasserstoff steht, und
**R²** für Wasserstoff oder Chlor steht, und
**R⁴** für Wasserstoff, Chlor oder Brom steht, und
**R³** für Wasserstoff steht, und
**Hal** für Fluor steht, und
**R⁵** für Wasserstoff und **R⁶** für eine Methylgruppe steht oder
**R⁵ und R⁶** zusammen mit dem Stickstoffatom der Seitenkette einen Pyrrolidinring bilden, und
**R^{17'}** für Wasserstoff steht, und
**R^{17"}** für einen Methylrest steht, und
**U** für einen n-Butylenrest steht, und
**V** für eine Methylengruppe steht, und
**X** für eine Bindung steht, und
**Y** für eine n-Pentylen- oder n-Hexylengruppe steht, und
**E** für -C₂F₅ steht,
sowie deren Enantiomere und Diastereomere, deren Salze, Solvate und Salze der Solvate.

14. Intermediate der Formel (IX) 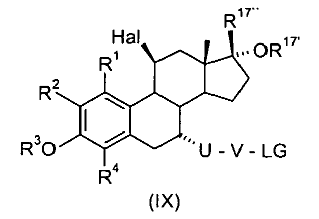 in der R¹, R². R³, R⁴, Hal, R^{17'}, R^{17"}, U und V die Bedeutungen gemäß einem der Ansprüche 1 bis 13 haben und LG für eine Sulfonatgruppe oder Halogen steht.

15. Verbindungen gemäß einem der Ansprüche 1 bis 13 zur Verwendung als Arzneimittel.

16. Verwendung einer Verbindung gemäß einem der Ansprüche 1 bis 13 zur Herstellung eines Medikamentes zur Behandlung von estrogen-abhängigen Erkrankungen.

17. Pharmazeutische Formulierung enthaltend eine Verbindung gemäß einem der Ansprüche 1 bis 13.
